# EUROPÄISCHE PATENTSCHRIFT

(11) **EP 2 873 426 B1**
(45) Veröffentlichungstag und Bekanntmachung des Hinweises auf die Patenterteilung: **21.06.2017**
(21) Anmeldenummer: 14191430.9
(22) Anmeldetag: 03.11.2014
(51) Int. Cl.: A61L 2/26, B01D 39/20

(54) **Dauerfilter für einen Sterilisationsbehälter, Sterilisationsbehälter und Verfahren zum Herstellen eines Dauerfilters**
Permanent filter for a sterilization container, sterilization container and method for producing a permanent filter
Filtre permanent pour un récipient de stérilisation, récipient de stérilisation et procédé de fabrication d'un filtre permanent

(30) Priorität: 05.11.2013 DE 102013112129
(43) Veröffentlichungstag der Anmeldung: 20.05.2015
(73) Patentinhaber: AESCULAP AG, 78532 Tuttlingen (DE)
(72) Erfinder: Weißhaupt, Dieter, 78194 Immendingen (DE); Weik, Thomas, 78532 Tuttlingen (DE); Schuster, Stefan, 78048 Villingen-Schwenningen (DE); Gray-Dreizler, John, 78628 Rottweil (DE); Burger, Wolfgang, 73207 Plochingen (DE)
(74) Vertreter: Hoeger, Stellrecht & Partner Patentanwälte mbB

(56) Entgegenhaltungen:
- EP-A1- 0 644 279
- WO-A1-01/08583
- DE-A1- 19 851 239
- US-A- 4 407 967

## Beschreibung

Die vorliegende Erfindung betrifft einen Dauerfilter für einen medizinischen Sterilisationsbehälter, wobei der Dauerfilter aus einer Keramik hergestellt ist und dass die Keramik aus globulären Substratkörnern hergestellt ist.

Ferner betrifft die vorliegende Erfindung einen medizinischen Sterilisationsbehälter, insbesondere zum Aufnehmen und Lagern zu sterilisierender Gegenstände, mit einem Behälterunterteil und einem Behälteroberteil zum Verschließen des Behälterunterteils in einer Schließstellung des Sterilisationsbehälters, wobei der Sterilisationsbehälter einen Behälterinnenraum definiert, welcher vom Behälterunterteil und vom Behälteroberteil begrenzt wird, und wobei der Behälterunterteil und/oder der Behälteroberteil eine Gasaustauschöffnung aufweisen, welche mit einem Dauerfilter verschlossen ist.

Ferner betrifft die vorliegende Erfindung ein Verfahren zum Herstellen eines Dauerfilters für einen medizinischen Sterilisationsbehälter, bei welchem der Dauerfilter durch Sintern aus einem keramischen Material hergestellt wird und als keramisches Material globuläre Substratkörner verwendet werden.

Sterilisationsbehälter mit Dauerfiltern aus einem Kunststoff, insbesondere Polytetrafluorethylen, sind beispielsweise aus der DE 298 19 825 U1 bekannt. Sie werden anstelle herkömmlicher Wegwerffilter, insbesondere Papierfilter, in Sterilisationsbehältern zum Verschließen von Gasaustauschöffnungen genutzt. Zwar erfüllen derartige Kunststofffilter die Anforderungen an die Porosität und die Porengröße. Allerdings zeigen sie Schwächen hinsichtlich der Re-Sterilisierbarkeit.

Aus der US 4,407,967 sind Verfahren zur Herstellung sphäroidaler Keramiken bekannt. In der EP 0 644 279 A1 ist α-Aluminium beschrieben. Sterilisierbehälter sind in der DE 198 51 239 A1 offenbart.

Es ist daher eine Aufgabe der vorliegenden Erfindung, einen Dauerfilter, einen Sterilisationsbehälter und ein Verfahren zum Herstellen eines Dauerfilters der eingangs beschriebenen Art so zu verbessern, dass die Nachteile bei der Re-Sterilisierbarkeit von Kunststofffiltern überwunden werden.

Diese Aufgabe wird bei einem Dauerfilter der eingangs beschriebenen Art erfindungsgemäß dadurch gelöst, dass der Dauerfilter mit einer hydrophoben Beschichtung versehen ist und dass die hydrophobe Beschichtung im Volumen des Dauerfilters aufgebracht ist.

Ein solcher keramischer Dauerfilter weist die gewünschte Bioinertheit sowie Biokompabilität auf, um insbesondere für medizinische Sterilisationsbehälter dienen zu können. Ferner ermöglichen es die globulären, also kugelförmigen oder im Wesentlichen kugelförmigen beziehungsweise kugeligen Substratkörner, eine Porosität sowie eine Porengröße des Dauerfilters in gewünschter Weise einstellen zu können. Globuläre Substratkörner haben im Gegensatz zu keramischen Substraten aus Pulvern oder anderen nicht globulären Körnern den Vorteil, dass die Porosität und damit auch die Durchlässigkeit des Dauerfilters deutlich besser eingestellt werden kann. Ein keramisches Dauerfilter ermöglicht insbesondere den Einsatz als bakteriologische Barriere und eine Sterilisierbarkeit in gesättigtem Wasserdampf. Bedingungen hierfür können insbesondere ein Druck von 4 bar und eine Sterilisationstemperatur von 143°C sein. Keramische Dauerfilter eignen sich zur Filtration von Wasserdampf und Luft, gestatten einen Durchfluss auf beiden Seiten des Filters und sind zudem keimdicht gemäß DIN 58953. Anders bei geschmolzenen Keramikmaterialien, die beispielsweise zur Ausbildung des Dauerfilters gesintert werden können, ergeben sich bei globulären Substratkörnern definierte Poren. In Abhängigkeit einer Größe der globulären Substratkörner kann so auch auf einfache Weise die Porosität eingestellt werden. Globuläre Substratkörner haben insbesondere den Vorteil, dass sie in relativ definierter Weise anordenbar sind und eine Porosität in Abhängigkeit eines Durchmessers der Substratkörner definiert eingestellt werden kann. Der Dauerfilter ist mit einer hydrophoben Beschichtung versehen. Auf diese Weise lässt sich verhindern, dass sich der Dauerfilter mit Wasser vollsaugt. Um zu verhindern, dass sich der Dauerfilter im Volumen mit Wasser vollsaugt, ist die hydrophobe Beschichtung im Volumen des Dauerfilters aufgebracht. Vorzugsweise ist der Dauerfilter selbsttragend ohne Trägerelement ausgebildet. Ein selbsttragender Dauerfilter ermöglicht es, die komplette Filterfläche zu nutzen. Ferner lassen sich so auch Verschmutzungen eines Trägers oder Trägermaterials vermeiden. Insgesamt ermöglicht ein selbsttragender Dauerfilter eine Minimierung sowohl der Anzahl als auch der Flächen von Gasaustauschöffnungen an Sterilisationsbehältern.
Vorteilhaft ist es, wenn die Substratkörner hergestellt sind durch Dispergieren und De-Agglomerieren von Keramikpulver in wässriger Suspension zur Erzeugung einzelner Primärkörner, Sprühtrocknen der die Primärkörner enthaltenden Suspension und Kalzinieren der Primärkörner zu Sekundärkörnern, welche die globulären Substratkörner bilden. Durch die Kalzination der Primärkörner zu Sekundärkörnern ist es insbesondere möglich, globuläre Substratkörner auszubilden. Insbesondere für einen Mindestdurchsatz an Luft und/oder Dampf durch den Dauerfilter ist eine bestimmte Durchlässigkeit erforderlich. Auf die beschriebene Weise lassen sich globuläre Substratkörner in gewünschter Größe und Qualität ausbilden.
Besonders vorteilhaft ist es, wenn das Keramikpulver Aluminiumoxid (Al₂O₃), Zirkonoxid (ZrO₂) Titandioxid (TiO₂) Mullit, Silikat, Kaolin oder eine beliebige Mischung derselben ist. Die genannten Materialien weisen die geforderten Eigenschaften hinsichtlich Bioinertheit und Biokompabilität auf, die die geforderte Re-Sterilisierbarkeit des Dauerfilters ermöglichen.

Günstigerweise ist das Keramikpulver γ-Aluminiumoxid (γ-Al₂O₃). Diese Aluminiumoxid-Modifikation lässt sich hervorragend als Ausgangsmaterial benutzen, da sie gezielt weiter bearbeitet beziehungsweise behandelt werden kann, insbesondere zur Ausbildung einer stabilen α-Aluminiumoxid-(α-Al₂O₃)-Modifikation.

Vorzugsweise sind die globulären Substratkörner durch das Kalzinieren von γ-Aluminiumoxid (γ-Al₂O₃) in α-Aluminiumoxid (α-Al₂O₃) hergestellt. Die Ausbildung der stabilen α-Aluminiumoxid-Modifikation lässt sich am einfachsten erreichen, wenn als Ausgangsmaterial γ-Aluminiumoxid verwendet wird. Zudem lassen sich so besonders stabile globuläre Substratkörner ausbilden.

Um eine möglichst hohe Qualität des gebildeten α-Aluminiumoxids sicherstellen zu können, ist es günstig, wenn die globulären Substratkörner durch Kalzinieren bei Temperaturen von mindestens etwa 1100°C hergestellt werden. Insbesondere ist es vorteilhaft, wenn die Kalzination bei einer Temperatur in einem Bereich von etwa 1300°C bis etwa 1500°C erfolgt. Günstig ist es, die Kalzination bei etwa 1350°C durchzuführen. Abhängig von der Temperatur ergibt sich insbesondere eine Mikroporosität der globulären Substratkörner. Je höher zum Beispiel die Temperatur, umso geringer die Mikroporosität. Beispielsweise können bei Kalzinationstemperaturen von mindestens 1400°C praktisch dichte, also unporöse, globuläre Substratkörner aus γ-Aluminiumoxid gebildet werden.

Zur Ausbildung eines dauerhaft stabilen Dauerfilters ist es vorteilhaft, wenn dieser durch Sintern bei Temperaturen im Bereich von etwa 1350°C bis etwa 1700°C hergestellt ist. Vorzugsweise wird bei Temperaturen in einem Bereich von etwa 1390°C bis etwa 1650°C gesintert. Temperaturen im angegebenen Bereich zur Herstellung der Dauerfilter zu benutzen, ermöglicht es, diese hinreichend stabil ausbilden zu können, insbesondere um eine selbsttragende Ausführung der Dauerfilter zu ermöglichen. Abhängig von der Sintertemperatur ergibt sich eine Mikroporosität der globulären Substratkörner. Je höher die Temperatur, umso geringer die Mikroporosität.

Vorteilhaft ist es, wenn der Dauerfilter durch Sintern mit einer Sinterzeit in einem Bereich von etwa 150 Minuten bis etwa 330 Minuten hergestellt ist. Vorzugsweise beträgt die Sinterzeit etwa 180 Minuten bis etwa 300 Minuten. Das Sintern der globulären Substratkörner für eine Sinterzeit in den angegebenen Bereichen stellt die erforderliche hohe Qualität der Dauerfilter sicher.

Um insbesondere die Festigkeit des Dauerfilters zu verbessern, ist es vorteilhaft, wenn der Dauerfilter hergestellt ist durch Zusetzen von Sinteradditiven zu den globulären Substratkörnern vor dem Sintern. Um die sogenannte Mikrokeramiken bildenden globulären Substratkörner in einen Haftverbund zu überführen, müssen diese gepresst und wie beschrieben bei sehr hohen Sintertemperaturen gesintert werden. Ein sehr guter Haftverbund kann aber insbesondere auch erreicht werden, wenn man den globulären Substratkörnern Sinteradditive zulegiert. Die Zugabe von Sinteradditiven ermöglicht die Ausbildung der Dauerfilter durch Sintern bei niedrigeren Temperaturen. Insbesondere kann dann in Abhängigkeit der gewählten Sinteradditive beispielsweise auch eine Farbe des Dauerfilters vorgegeben werden.

Günstig ist es, wenn der Dauerfilter hergestellt ist mit Sinteradditiven in Form von sinteraktivem Aluminiumoxidpulver, vorzugsweise etwa 10 bis etwa 30 Gewichtsprozent, und/oder sinteraktivem Titanoxid, Magnesiumoxid, Siliziumoxid, Eisenoxid, Manganoxid, Nickeloxid, Cobaltoxid, Chromoxid und/oder Seltenerdoxiden. Die Zugabe eines oder mehrerer der genannten Materialien ermöglicht die Ausbildung eines optimalen Haftverbunds der globulären Substratkörner zu einem stabilen Dauerfilter.

Besonders günstig ist es, wenn der Dauerfilter hergestellt ist durch Einsetzen von etwa 1 bis 1,5 Gewichtsprozent Titanoxid (TiO₂) und/oder etwa 0,2 Gewichtsprozent Magnesiumoxid (MgO). Eine solche Zugabe an Sinteradditiven ermöglicht die Ausbildung eines Dauerfilters mit den gewünschten Eigenschaften.

Vorzugsweise enthält der Dauerfilter einen Anteil globulärer Substratkörner von etwa 75 bis etwa 85 Gewichtsprozent und einen Anteil von Sinteradditiven von etwa 15 bis etwa 25 Gewichtsprozent. Derart ausgebildete Dauerfilter weisen eine sehr gute und homogene Porosität auf.

Die Stabilität des Dauerfilters lässt sich weiter verbessern, wenn der Dauerfilter hergestellt ist durch Mahlen der globulären Substratkörner vor dem Sintern. Insbesondere kann das Mahlen nach dem Zusetzen eines Sinteradditives erfolgen. Auf diese Weise lässt sich eine optimale Verbindung der globulären Substratkörner beim Sintern sicherstellen.

Insbesondere ist es günstig, wenn der Dauerfilter hergestellt ist durch Zusetzen eines Bindemittels nach dem Mahlen der Mischung aus den globulären Substratkörnern und dem Sinteradditiv. So kann ein Zusammenhalt, also eine primäre Formstabilität des Filterrohlings, erreicht werden.

Gemäß einer weiteren bevorzugten Ausführungsform der Erfindung kann vorgesehen sein, dass der Dauerfilter durch in Form Bringen der Mischung nach dem Zusetzen des Bindemittels in einem Presswerkzeug, Entfernen des Bindemittels, vorzugsweise thermisch, und anschließendes Sintern hergestellt ist. Ein derart gepresster, mit dem Bindemittel vorgebundener Rohling, der durch Sintern in seine endgültige Form und Mikrokonfiguration gebracht wird, weist in reproduzierbarer Weise die gewünschten Filtereigenschaften auf.

Vorteilhaft ist es, wenn der Dauerfilter durch eine Fraktionierung der sprühgetrockneten Primärkörner durch Sieben hergestellt ist. Dies hat den Vorteil, dass praktisch nur Primärkörner einer vorgegebenen definierten Größe für die Kalzination und den anschließenden Sinterprozess verwendet werden. So kann eine homogene Verteilung gleich großer oder im Wesentlichen gleich großer globulärer Substratkörner erreicht werden, was eine definierte Porosität für den Dauerfilter gewährleistet.

Für einen Dauerfilter eines Sterilisationsbehälters erforderliche Durchflussraten lassen sich insbesondere erreichen, wenn Korngrößen der durch das Sieben fraktionierten Primärkornfraktion einen Korndurchmesser in einem Bereich von etwa 90 µm bis etwa 150 µm aufweisen.

Die Qualität des Dauerfilters lässt sich weiter verbessern, wenn er hergestellt ist durch Sprühtrocknen der Suspension unter Zugabe eines organischen Bindemittels. Insbesondere ist es günstig, wenn das organische Bindemittel Polyvinylalkohol oder Polyacrylat ist.

Insbesondere wenn das Keramikpulver Zirkonoxid ist, ist es günstig, wenn dieses mit MgO, CaO, Y₂O₃, CeO₂ oder Mischungen aus den genannten Verbindungen stabilisiert ist.

Vorteilhaft ist es, wenn der Dauerfilter mit einer hydrophoben Beschichtung versehen ist. Auf diese Weise lässt sich verhindern, dass sich der Dauerfilter mit Wasser vollsaugt.

Eine gute wasserabweisende Wirkung lässt sich beispielsweise dadurch erreichen, dass die hydrophobe Beschichtung eine Oberfläche des Dauerfilters bedeckt.

Um zu verhindern, dass sich der Dauerfilter im Volumen mit Wasser vollsaugt, ist es günstig, wenn die hydrophobe Beschichtung im Volumen des Dauerfilters aufgebracht ist.

Auf einfache und kostengünstige Weise lässt sich eine hydrophobe Beschichtung des Dauerfilters realisieren, wenn die hydrophobe Beschichtung Siloxan und/oder Teflon enthält oder ist. Insbesondere ist eine Teflon enthaltende oder aus Teflon bestehende Beschichtung auch bei der Anwendung von Reinigungsmitteln mit einem pH-Wert größer als 10 stabil.

Die eingangs gestellte Aufgabe wird ferner bei einem medizinischen Sterilisationsbehälter der eingangs beschriebenen Art erfindungsgemäß dadurch gelöst, dass er einen der oben beschriebenen Dauerfilter umfasst.

Ein Sterilisationsbehälter mit einem der oben beschriebenen keramischen Dauerfilter ermöglicht die sichere, luftdichte und keimdichte Lagerung von Gegenständen, insbesondere Implantaten und chirurgischen Instrumenten im Behälterinnenraum. Zudem ist ein Filterwechsel nicht erforderlich. Der keramische Dauerfilter ermöglicht es zudem, selbst sterilisiert zu werden.

Die eingangs gestellte Aufgabe wird ferner bei einem Verfahren der eingangs beschriebenen Art erfindungsgemäß dadurch gelöst, dass der Dauerfilter mit einer hydrophoben Beschichtung versehen wird und dass die hydrophobe Beschichtung im Volumen des Dauerfilters aufgebracht wird.

Auf diese Weise lassen sich keramische Dauerfilter herstellen, die hinreichend bioinert und biokompatibel sind, um insbesondere als Filter für medizinische Sterilisationsbehälter dauerhaft dienen zu können. Außerdem kann so eine Porosität sowie eine Porengröße des Dauerfilters in definierter Weise eingestellt werden. Ferner ist die Handhabung globulärer Substratkörner relativ einfach. Zudem können die Sterilisationsbedingungen wie oben beschrieben durch ein solches keramisches Dauerfilter ohne Schwierigkeiten erfüllt werden. Insgesamt kann ein homogener Filter ausgebildet werden, also mit nur geringen Unterschieden bei den von ihm definierten Poren hinsichtlich deren Größe und Form. Der Dauerfilter wird mit einer hydrophoben Beschichtung versehen. Auf diese Weise lässt sich verhindern, dass sich der Dauerfilter mit Wasser vollsaugt. Um zu verhindern, dass sich der Dauerfilter im Volumen mit Wasser vollsaugt, wird die hydrophobe Beschichtung im Volumen des Dauerfilters aufgebracht.

Günstig ist es, wenn die Substratkörner hergestellt werden durch Dispergieren und De-Agglomerieren von Keramikpulver in wässriger Suspension zur Erzeugung einzelner Primärkörner, Sprühtrocknen der die Primärkörner enthaltenden Suspension und Kalzinieren der Primärkörner zu Sekundärkörnern, welche die globulären Substratkörner bilden. Insbesondere die Kalzination ermöglicht die Ausbildung globulärer Substratkörner definierter Größe. Da für den Mindestdurchsatz an Luft und Heißdampf durch den Dauerfilter eine bestimmte Durchlässigkeit erforderlich ist, können die hierfür benötigten Sekundärkörner in gewünschter Größe auf einfache und reproduzierbare Weise hergestellt werden.

Vorteilhaft ist es, wenn als Keramikpulver Aluminiumoxid (Al₂O₃), Zirkonoxid (ZrO₂), Titandioxid (TiO₂) Mullit, Silikat, Kaolin oder eine beliebige Mischung derselben eingesetzt wird. Diese Materialen weisen gewünschte Eigenschaften im Hinblick auf die für den Dauerfilter geforderte Bioinertheit und Biokompabilität auf, um so eine Sterilisierbarkeit des Dauerfilters zu ermöglichen.

Des Weiteren ist es vorteilhaft, wenn Aluminiumoxid (Al₂O₃) in Form von γ-Aluminiumoxid (γ-Al₂O₃) verwendet wird. Aus diesem Ausgangsmaterial lässt sich in einer nächsten Verfahrensschritt das besonders stabile γ-Aluminiumoxid (γ-Al₂O₃) ausbilden. Diese Aluminiumoxid-Modifikation ist insbesondere zur Ausbildung des Dauerfilters hoch stabil.

Ferner ist es günstig, wenn das Kalzinieren bei Temperaturen von mindestens etwa 1100°C erfolgt. Insbesondere ist es vorteilhaft, wenn das Kalzinieren in einem Temperaturbereich von etwa 1300°C bis etwa 1500°C erfolgt. Günstig ist es, wenn das Kalzinieren bei etwa 1350°C durchgeführt wird. Eine Kalzination bei diesen Temperaturen durchzuführen ermöglicht es, α-Aluminiumoxid in hoher Qualität bilden zu können.

Um einen dauerhaft stabilen Dauerfilter auszubilden, ist es günstig, wenn hierfür globuläre Substratkörner bei Temperaturen im Bereich von etwa 1350°C bis etwa 1700°C gesintert werden. Günstig ist es, wenn das Sintern in einem Temperaturbereich von etwa 1390°C bis etwa 1650°C erfolgt. Bei diesen Temperaturen zu sintern erlaubt es, hinreichend stabile Dauerfilter auszubilden, insbesondere können so selbsttragende Dauerfilter zuverlässig realisiert werden.

Vorzugsweise erfolgt dass Sintern für eine Sinterzeit in einem Bereich von etwa 150 Minuten bis etwa 330 Minuten. Günstig ist es, wenn die Sinterzeit etwa 180 Minuten bis etwa 300 Minuten beträgt. Auf diese Weise lassen sich Dauerfilter mit hoher Qualität herstellen.

Zur Verbesserung der Stabilität und Festigkeit des Dauerfilters ist es günstig, wenn den globulären Substratkörnern vor dem Sintern Sinteradditive zugesetzt werden. Sinteradditive verbessern einen Haftverbund der globulären Substratkörner. Zudem sind beim Einsatz von Sinteradditiven niedrigere Sintertemperaturen ausreichend, um ein dauerhaft stabiles Dauerfilter auszubilden.

Günstig ist es, wenn als Sinteradditive sinteraktives Aluminiumoxidpulver, vorzugsweise etwa 10 bis etwa 30 Gewichtsprozent, und/oder sinteraktives Titanoxid, Magnesiumoxid, Siliziumoxid, Eisenoxid, Manganoxid, Nickeloxid, Cobaltoxid, Chromoxid und/oder Seltenerdoxide eingesetzt werden. Durch Zugabe eines oder mehrerer dieser Materialien kann ein optimaler Haftverbund der globulären Substratkörper ausgebildet werden zur Herstellung eines stabilen Dauerfilters. Insbesondere Chromoxid sowie Seltenerdoxide ermöglichen es, dem Dauerfilter eine gewünschte Farbe zu geben. Damit kann das Dauerfilter insbesondere bei Bedarf dauerhaft gekennzeichnet werden.

Vorteilhafterweise werden etwa 1 bis 1,5 Gewichtsprozent Titanoxid (TiO₂) und/oder etwa 0,2 Gewichtsprozent Magnesiumoxid (MgO) eingesetzt. Diese Sinteradditive zuzugeben ermöglicht es, einen Dauerfilter mit den benötigten Eigenschaften herzustellen.

Gemäß einer weiteren bevorzugten Variante des erfindungsgemäßen Verfahrens ist es günstig, wenn ein Anteil globulärer Substratkörner nach dem Sintern etwa 75 bis etwa 85 Gewichtsprozent beträgt und ein Anteil der Sinteradditive etwa 15 bis etwa 25 Gewichtsprozent. Dauerfilter dieser Zusammensetzung weisen eine sehr gute und homogene Porosität auf. Sie werden im Wesentlichen bestimmt durch die gewählten globulären Substratkörner, deren Eigenschaften durch die Sinteradditive nur begrenzt überdeckt werden.

Günstigerweise werden die globulären Substratkörner vor dem Sintern, insbesondere nach dem Zusetzen eines Sinteradditivs, gemahlen. So kann eine optimale Verbindung der globulären Substratkörner beim Sintern erreicht werden.

Zum Verbessern der Stabilität des Filters ist es vorteilhaft, wenn nach dem Mahlen der Mischung aus den globulären Substratkörnern und dem Sinteradditiv mindestens ein Bindemittel zugesetzt wird. So kann vor dem Sintern mittels eines Presswerkzeugs ein Filterrohling hergestellt werden, der bereits gut zusammenhält. Anschließendes Entfernen des Bindemittels, vorzugsweise thermisch, und danach Sintern des Filterrohlings erlaubt es, Dauerfilter mit den gewünschten Eigenschaften auszubilden.

Gemäß einer weiteren bevorzugten Variante des erfindungsgemäßen Verfahrens kann vorgesehen sein, dass die Mischung nach dem Zusetzen des Bindemittels in einem Presswerkzeug in Form gebracht, das mindestens eine Bindemittel entfernt, vorzugsweise thermisch, und gesintert wird. Bei dem Presswerkzeug kann die Mischung aus globulären Substratkörnern und dem Bindemittel in eine Form gebracht werden zur Ausbildung eines Filterrohlings. Das Bindemittel kann anschließend entfernt werden, beispielsweise durch Aufheizen, und durch Sintern kann der Filterrohling in die endgültige Form des Dauerfilters mit der gewünschten Mikrokonfiguration an Poren erzeugt werden.

Vorzugsweise erfolgt eine Fraktionierung der sprühgetrockneten Primärkörner durch Sieben. So können auf einfache Weise aus den sprühgetrockneten Primärkörnern diejenigen ausgewählt werden, die eine vorgegebene definierte Größe haben, um nach der Kalzination und dem anschließenden Sinterprozess Poren des Dauerfilters in gewünschter Größe und Anzahl zu bilden.

Vorteilhaft ist es, wenn durch das Sieben eine Primärkornfraktion mit einem Korndurchmesser in einem Bereich von etwa 90 µm bis etwa 150 µm fraktioniert wird. Auf diese Weise können die für einen Sterilisationsbehälter erforderlichen Durchflussraten vorgegeben werden.

Das Sprühtrocknen lässt sich auf einfache Weise durchführen und eine Qualität des Dauerfilters verbessern, wenn das Sprühtrocknen der Suspension unter Zugabe eines organischen Bindemittels erfolgt. Insbesondere sind Polyvinylalkohol oder Polyacrylat als organische Bindemittel geeignet.

Zur Herstellung dauerhaft stabiler Dauerfilter aus Zirkonoxid ist es günstig, wenn das Zirkonoxid mit MgO, CaO, Y₂O₃, CeO₂ oder Mischungen aus den genannten Verbindungen stabilisiert wird.

Die Herstellung des Dauerfilters lässt sich weiter vereinfachen und kostengünstiger gestalten, wenn der Dauerfilter selbsttragend ohne Trägerelement hergestellt wird.

Eine gute wasserabweisende Wirkung lässt sich beispielsweise dadurch erreichen, dass die hydrophobe Beschichtung eine Oberfläche des Dauerfilters bedeckt wird.

Auf einfache und kostengünstige Weise lässt sich eine hydrophobe Beschichtung des Dauerfilters realisieren, wenn die hydrophobe Beschichtung durch Aufbringen von Siloxan und/oder Teflon ausgebildet wird. Insbesondere ist eine Teflon enthaltende oder aus Teflon bestehende Beschichtung auch bei der Anwendung von Reinigungsmitteln mit einem pH-Wert größer als 10 stabil.

Die nachfolgende Beschreibung bevorzugter Ausführungsformen der Erfindungen dienen im Zusammenhang mit den Zeichnungen der näheren Erläuterung. Es zeigen:
- Figur 1:: eine schematische Darstellung eines ersten Ausführungsbeispiels eines Sterilisationsbehälters mit einem Behälterunterteil und einem Behälteroberteil und einem Dauerfilter;
- Figur 2:: eine perspektivische schematische Darstellung eines zweiten Ausführungsbeispiels eines Sterilisationsbehälters mit einem Dauerfilter;

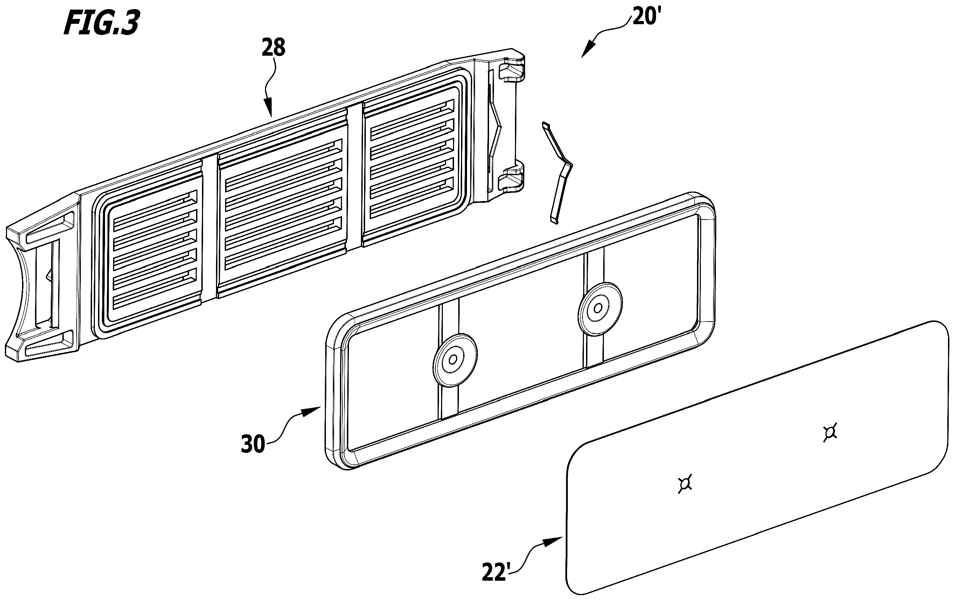

- Figur 3:: eine schematische Explosionsdarstellung der in Figur 2 dargestellten Filteranordnung mit einem keramischen Dauerfilter;
- Figur 4:: γ-Al₂O₃ (1390 °C, links), α-Al₂O₃ (1390 °C, rechts);
- Figur 5:: γ-Al₂O₃ (1500 °C, links); α-Al₂O₃ (1500 °C, rechts);
- Figur 6:: γ-Al₂O₃ (1650 °C, links); α-Al₂O₃ (1650 °C, rechts);
- Figur 7:: REM-Bilder von Oberflächen einer Keramik aus Aluminiumoxidkörnern, sogenanntem Schmelzkorund, in den angegebenen Vergrößerungen;
- Figur 8:: REM-Bilder von Bruchflächen der Keramik aus Figur 7;
- Figur 9:: REM-Bilder bei einer Kalzinationstemperatur T_{calc} = 1300 °C (oben) und T_{calc} = 1450 °C (unten);
- Figur 10:: REM-Bruch-Bilder der Mikrostruktur eines Dauerfilter;
- Figur 11:: REM-Bilder an V0500;
- Figur 12:: REM-Aufnahmen an Mustern aus dem Presswerkzeug;
- Figur 13:: Kornverteilung der Siebfraktionen i) 150 - 90 µm (links) und ii) 106 - 90 µm (rechts);
- Figur 14:: Siebfraktion 150 - 90 µm; links vor Kalzination und rechts nach Kalzination;
- Figur 15:: Siebfraktion 106 - 90 µm; links vor Kalzination und rechts nach Kalzination;
- Figur 16:: Kalzinat ohne (links) und mit (rechts) Tonerde;
- Figur 17:: V0453-Oberfläche (1450°C/5h);
- Figur 18:: V0453-Bruch (1525°C/5h);
- Figur 19:: V0458-Oberfläche (1580°C/5h);
- Figur 20:: V0458-Bruch (1580°C/5h);
- Figur 21:: V0459-Oberfläche (1580°C/5h);
- Figur 22:: V0459-Bruch (1580°C/5h);
- Figur 23:: V0480-Oberfläche (1600°C/5h);
- Figur 24:: V0480-Bruch (1600°C/5h); und
- Figur 25:: V0480-Bruch (1450°C/5h).

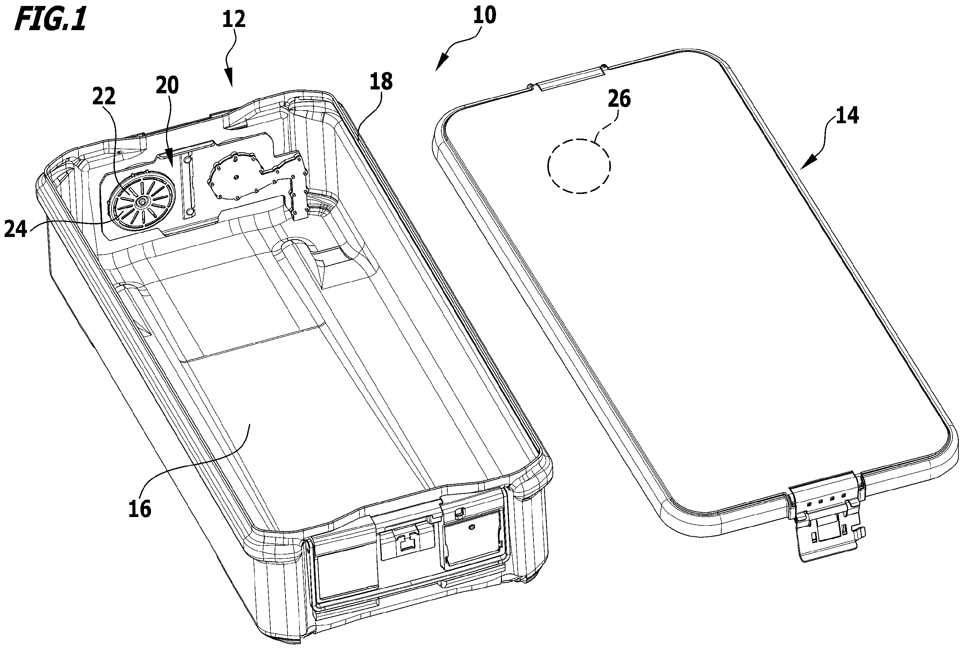

In Figur 1 ist schematisch ein Sterilisationsbehälter 10 dargestellt, welcher ein wannenförmiges Behälterunterteil 12 und ein in Form eines Deckels ausgebildetes Behälteroberteil 14 umfasst. Das Behälteroberteil 14 ist ausgebildet zum Verschließen des Behälterunterteils 12 in einer nicht dargestellten Schließstellung des Sterilisationsbehälters 10. Der Sterilisationsbehälter 10 definiert einen Behälterinnenraum 16, welcher vom Behälterunterteil 12 und vom Behälteroberteil 14 begrenzt wird. Diese sind in der Schließstellung gegeneinander abgedichtet, beispielsweise durch eine auf einer Unterseite des Behälteroberteils 14 angeordnete Dichtung, die auf einer umlaufenden Kante 18 des Behälterunterteils 12 aufliegt.

Am Behälterunterteil 12 ist schematisch in Figur 1 dargestellt eine Filteranordnung 20 angeordnet, die einen scheibenförmigen Dauerfilter 22 umfasst, welcher in der Filteranordnung 20 von einer Abdeckung 24 verdeckt ist. Der Dauerfilter 22 ist in Form eines keramischen Dauerfilters ausgebildet, dessen Aufbau und Herstellung nachfolgend noch im Einzelnen beschrieben wird. Die Filteranordnung 20 ist so ausgebildet und der Dauerfilter 22 in dieser derart angeordnet, dass eine nicht näher dargestellte, in Form einer Durchbrechung ausgebildete Gasaustauschöffnung am Behälterunterteil 12 vollständig durch den Dauerfilter 22 abgedeckt und verschlossen wird. Dadurch ist ein Gasaustausch zwischen einer Umgebung des Sterilisationsbehälters 10 und dem Behälterinnenraum 16 in der Schließstellung nur durch den Dauerfilter 22 hindurch möglich.

Alternativ oder zusätzlich kann, wie schematisch in Figur 1 am Behälteroberteil gestrichelt dargestellt, auch eine Gasaustauschöffnung 26 am Behälteroberteil 14 angeordnet oder ausgebildet sein, die dann vom Dauerfilter 22 verschlossen werden kann, beispielsweise mit einer den Dauerfilter 22 umfassenden Filteranordnung 20.

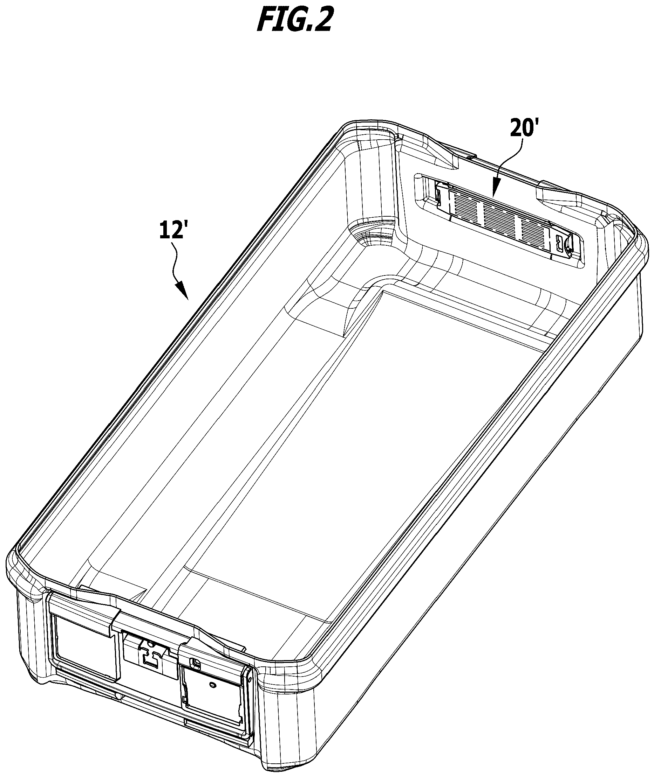

In Figur 2 ist schematisch eine zweite Variante eines Behälterunterteils 12' dargestellt. An einer Stirnseite derselben ist eine Variante einer Filteranordnung 20' angeordnet, die eine im Wesentlichen rechteckige Gasaustauschöffnung verschließt. Die Filteranordnung 20' umfasst eine Abdeckung 28, eine Halterahmen 30 und einen im Wesentlichen rechteckigen Dauerfilter 22', welcher durchbrechungsfrei ausgebildet ist. Er kann zwischen der Abdeckung 28 und dem Halterahmen 30 klemmend gehalten werden.

Die Dauerfilter 22 und 22' werden durch Sintern aus einem keramischen Material hergestellt. Als keramisches Material werden globuläre Substratkörner verwendet. Diese werden durch Dispergieren und De-Agglomerieren eines Keramikpulvers in wässriger Suspension zur Erzeugung einzelner Primärkörner hergestellt. Die die Primärkörner enthaltende Suspension wird sprühgetrocknet und anschließend werden die Primärkörner zu Sekundärkörnern kalziniert. Die kalzinierten Sekundärkörner bilden dann die globulären Substratkörner. Diese sind kugelförmig oder im Wesentlichen kugelförmig beziehungsweise kugelig.

Als Keramikpulver wird Aluminiumoxid (Al₂O₃), Zirkonoxid (ZrO₂), Titandioxid (TiO₂) Mullit, Silikat, Kaolin oder eine beliebige Mischung derselben verwendet.

Bevorzugt wird als Aluminiumoxid (Al₂O₃) γ-Aluminiumoxid (γ-Al₂O₃) verwendet. Durch das Kalzinieren wird γ-Aluminiumoxid (γ-Al₂O₃) in α-Aluminiumoxid (α-Al₂O₃)umgewandelt. Diese Aluminiumoxid-Modifikation ist deutlich stabiler als die γ-Aluminiumoxid-Modifikation. Eine Umwandlung von γ-Aluminiumoxid in α-Aluminiumoxid erfolgt bei etwa 1050°C. Mit dieser Phasenumwandlung des Ausgangsmaterials ist jedoch eine Volumenschrumpfung verbunden. Diese Volumenschrumpfung, also die Verkleinerung der Primärkörner zu den Sekundärkörnern definiert dann letztlich im Verbund die Porosität des Dauerfilters.

Die Kalzination zur Herstellung des Dauerfilters erfolgt vorzugsweise bei Temperaturen von mindestens etwa 1100°C. Vorteilhaft ist es, wenn das Kalzinieren in einem Temperaturbereich von etwa 1300°C bis etwa 1500°C erfolgt. Günstigerweise wird bei etwa 1350°C kalziniert.

Optional können die sprühgetrockneten Primärkörner durch Sieben fraktioniert werden. Idealerweise wird durch Sieben eine Primärkornfraktion mit einem Korndurchmesser in einem Bereich von etwa 90 µm bis etwa 150 µm ausgesondert, die dann zur weiteren Herstellung des Dauerfilters verwendet werden.

Insbesondere erfolgt das Sprühtrocknen der wässrigen Suspension unter Zugabe eines organischen Bindemittels. Beispielsweise können hierfür Polyvinylalkohol oder Polyacrylat verwendet werden.

Zur abschließenden Formgebung und Ausbildung des Dauerfilters in praktisch beliebiger Form wird die Mischung der globulären Substratkörner vorzugsweise zunächst gemahlen. Zudem können auch die globulären Substratkörner vor einem Sinterprozess gemahlen werden.

Der Mahlschritt erfolgt vorzugsweise nach dem Zusetzen eines Sinteradditivs. Den globulären Substratkörnern werden optional vor dem Sintern Sinteradditive zugesetzt. Als Sinteradditive eignen sich insbesondere sinteraktives Aluminiumoxidpulver, vorzugsweise etwa 10 bis etwa 30 Gewichtsprozent. Alternativ oder zusätzlich können auch sinteraktives Titanoxid, Magnesiumoxid, Siliziumoxid, Eisenoxid, Manganoxid, Nickeloxid, Kobaltoxid, Chromoxid und/oder Seltenerdoxide als Sinteradditive eingesetzt werden. Beispielsweise werden 1 bis 1,5 Gewichtsprozent Titanoxid (TiO₂) und/oder etwa 0,2 Gewichtsprozent Magnesiumoxid (MgO) den globulären Substratkörnern als Sinteradditive zugesetzt.

Zur Ausbildung des Dauerfilters wird vorzugsweise eine Mischung aus globulären Substratkörnern und Sinteradditiven genutzt, wobei ein Anteil der globulären Substratkörner nach dem Sintern etwa 75 bis etwa 85 Gewichtsprozent beträgt, ein Anteil der Sinteradditive etwa 15 bis etwa 25 Gewichtsprozent.

Um ein Formen des Dauerfilters zu erleichtern und einem Filterrohling eine Primärstabilität zu verleihen, wird nach dem Mahlen der Mischung aus den globulären Substratkörnern und dem Sinteradditiv mindestens ein Bindemittel zugesetzt. Dies kann ein herkömmliches Bindemittel zur Ausbildung von Oxidkeramiken sein, welches thermisch entfernbar ist.

Die Mischung aus globulären Substratkörnern, Bindemittel und Sinteradditiven wird vorzugsweise in einem Presswerkzeug in Form gebracht werden. Danach kann das Bindemittel entfernt werden, beispielsweise thermisch durch Erhitzen des Filterrohlings.

In einem letzten Schritt wird der Filterrohling gesintert. Der Sinterprozess erfolgt vorzugsweise bei Temperaturen von etwa 1350°C bis etwa 1700°C. Dauerfilter hoher Qualität können insbesondere bei Sintertemperaturen in einem Bereich von etwa 1390°C bis etwa 1650°C erhalten werden.

Eine Sinterzeit liegt bevorzugt in einem Bereich von etwa 150 Minuten bis etwa 330 Minuten. Insbesondere kann die Sinterzeit etwa 180 Minuten bis etwa 300 Minuten betragen.

Anstelle von Aluminiumoxid kann als Keramikpulver zur Herstellung eines Dauerfilters auch Zirkonoxid verwendet werden. Zur Stabilisierung werden vorzugsweise MgO, CaO, Y₂O₃, CeO₂ oder Mischungen aus den genannten Verbindungen verwendet.

Nachfolgend werden konkrete Beispiele zur Ausbildung poröser keramischer Dauerfilter erörtert.

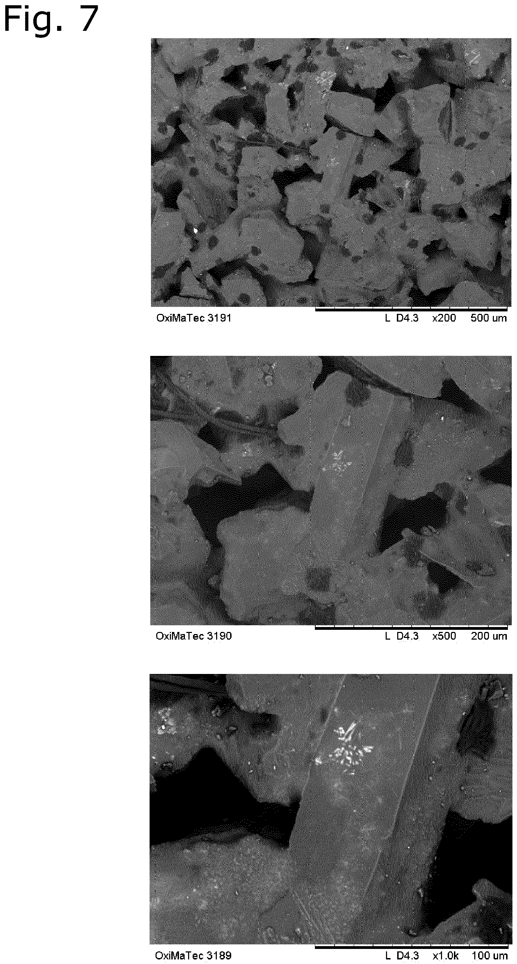

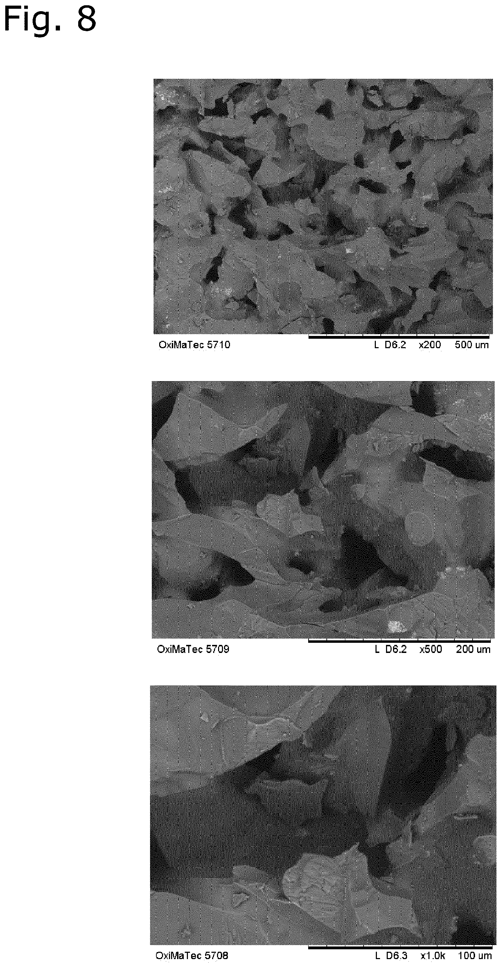

Keramiken aus Aluminiumoxidkörnern, sogenanntem Schmelzkorund, weisen eine weitestgehend irreguläre Struktur auf, wie dies in den REM-Bildern der Figuren 7 und 8 gut zu erkennen ist. Figur 7 zeigt Oberflächen, Figur 8 Bruchflächen solcher Keramiken in den angegebenen Vergrößerungen.

### Ausführungsbeispiel A:

Die globularen Körner des Dauerfilters werden wie folgt hergestellt:
- Dispergieren und De-Agglomerieren von Keramikpulver in wässriger Suspension;
- Sprühtrocknen der Suspension unter Zugabe eines organischen Bindemittels, beispielsweise Polyvinylalkohol oder Polyacrylat;
- Thermische Behandlung des Sprühkorns bei hohen Temperaturen zwecks Herstellung einer sogenannten Mikrokeramik in Form globulärer Substratkörner.

Als besonders geeignet hat sich dabei die Verwendung von Aluminiumoxidpulver herausgestellt. Dieses kann entweder γ-Al₂O₃ oder α-Al₂O₃ sein. Aber auch ZrO₂ (stabilisiert mit MgO, CaO, Y₂O₃, CeO₂ oder Mischungen daraus), TiO₂ Mullit bzw. andere Al₂O₃-SiO₂-Verbindungen (Silikate, Kaoline oder dergleichen, können als globuläre Substratkörner eingesetzt werden.

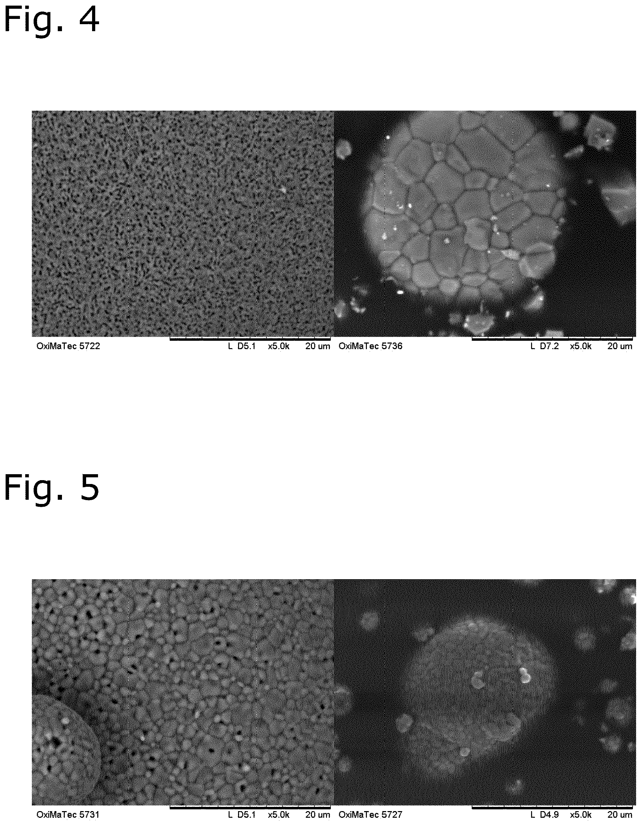

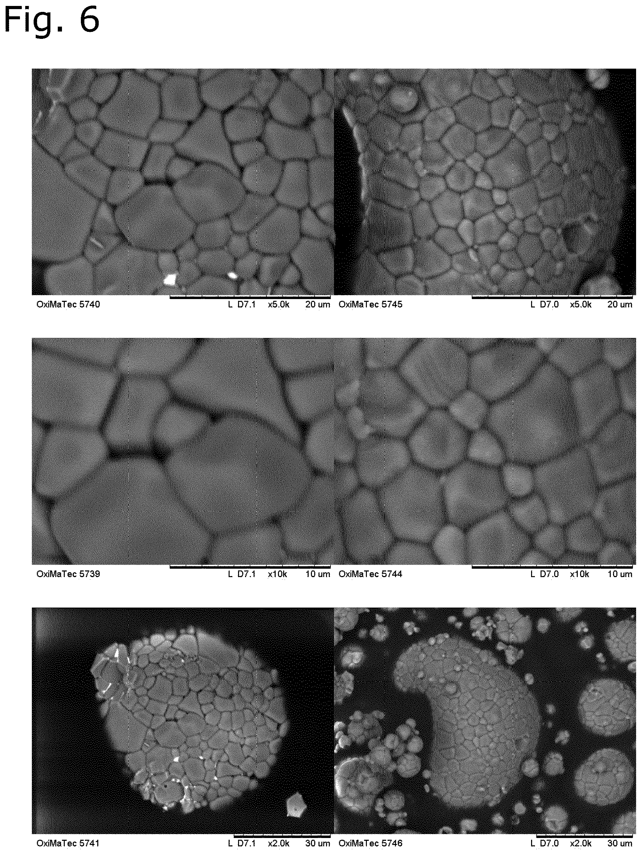

Bei diesem Ausführungsbeispiel basiert der Dauerfilter auf gesinterten Aluminiumoxidkörnern als Mikrokeramiken. Setzt man γ-Al₂O₃ ein, so entsteht während der Kalzination bei 1350 °C beziehungsweise 1450 °C eine deutlich höher zerklüftete Oberfläche mit entsprechender Porosität, als dies bei der Kalzination unter gleichen Bedingungen für das α-Al₂O₃ der Fall ist. Selbst bei einer Sintertemperatur von 1650 °C kann man zwischen den Körnern des kalzinierten γ-Al₂O₃ noch feinste Poren erkennen. Die Figuren 4 bis 6 zeigen REM-Bilder, welche die Unterschiede in der Mikrostruktur zeigen. Die jeweiligen Parameter der REM-Bilder sind unter denselben angegeben.

Um die Mikrokeramiken in einen Haftverbund zu überführen, werden diese gepresst und bei extrem hohen Sintertemperaturen gesintert. Ein sehr guter Haftverbund wird aber auch erreicht, wenn man den Mikrokeramiken sogenannte Sinteradditive zulegiert. Besonders bevorzugt werden arteigene Sinteradditive: Aluminiumoxid-Mikrokeramiken werden vorzugsweise mit sinteraktivem Aluminiumoxidpulver versetzt (10 - 30 wt-%) und auf der Oberfläche der Mikrokeramiken niedergeschlagen, damit der Haftverbund über die Ausbildung von Sinterhälsen auch bereits bei niedrigeren Temperaturen sicher gestellt wird.

Zusätzliche Sinteraktivität erreicht man über die Zulegierung von sinteraktivem Titanoxid, Magnesiumoxid, Siliciumdioxid. Aber auch Eisenoxid, Manganoxid, Nickeloxid, Cobaltoxid, Chromoxid und Seltenerdoxide können als Sinterhilfsmittel eingesetzt werden. Letztere führen teilweise auch zu einer anderen Farbe während des Sinterprozesses.

Die Zulegierung von Sinteradditiven zu den globulären Mikrokeramiken führt zu einer Senkung der Sintertemperatur und sorgt gleichzeitig für einen guten Haftverbund. Über deren Konzentration kann einerseits die Sintertemperatur und andererseits auch die Gesamtporosität des Filters beeinflusst werden. Als weitere Einflussgröße auf die Porosität und die damit verbundene Luftdurchlässigkeit ist die Größe der gesinterten globularen Körner zu nennen. Grundsätzlich gilt: je weniger Sinterhilfsmittel verfügbar sind und je größer die globulären Mikrokeramiken sind, desto höher wird die Durchlässigkeit des Filters und desto höhere Sintertemperaturen sind anzuwenden. Relativ kleine Mikrokeramiken sorgen im Keramikverbund für kleinere Poren und reduzieren somit den Luftdurchfluss.

### Ausführungsbeispiel B:

Keramiken aus Aluminiumoxidkörnern, sogenanntem Schmelzkorund, weisen eine weitestgehend irreguläre Struktur auf, wie dies in den in den REM-Bildern der Figuren 7 und 8 gut zu erkennen ist. Figur 7 zeigt die Oberfläche, Figur 8 Bruchflächen solcher Keramiken in den angegebenen Vergrößerungen.

Im Gegensatz hierzu basiert der strukturelle Aufbau in der Gefügestruktur im Wesentlichen auf kugelförmigen polykristallinen Partikeln. Diese kugelförmigen polykristallinen Partikel werden in einem ersten verfahrenstechnischen Schritt wie folgt hergestellt:
- Dispergieren und De-Aggomerieren von hochreinem γ-Al₂O₃;
- Hinzufügen von Bindemittel und Sprühtrocknen;
- Fraktionieren der Sprühgranulate und Kalzinieren bei einer Temperatur > 1100°C, damit das thermodynamisch stabile α-Al₂O₃ entsteht.

Über den Sprühtrocknungsprozess kann die Agglomeratgröße beeinflusst werden: verwendet man beispielsweise im Prozess eine Zweistoffdüse, so entstehen vorzugsweise Sekundärkörner zwischen 30 - 100 µm. Grobe Sekundärkörner erhält man über die Verwendung einer Druckdüse beim Sprühtrocknungsprozess.

Beim Sprühtrocknungsprozess fällt stets ein Produkt mit einer gewissen Kornverteilung an. Daher kann im Anschluss daran über die Anwendung eines Siebprozesses die weitere Einengung der gewünschten Fraktion erreicht werden.

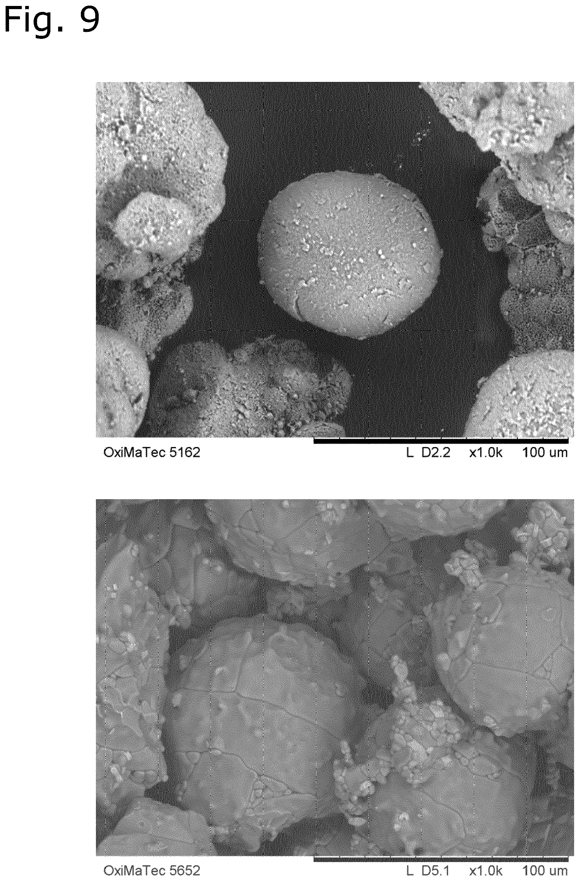

Unterzieht man die fraktionierten Sekundärkörner aus γ-Al₂O₃ einer thermischen Behandlung unter Anwendung einer Temperatur von mindestens 1100°C, so entsteht die thermodynamisch stabile α-Modifikation des Aluminiumoxids. Je höher die Kalzinationstemperatur gewählt wird, desto geringer wird die Oberflächenporosität der kugelförmigen polykristallinen Partikel. Die in Figur 9 dargestellten REM-Bilder bei einer Kalzinationstemperatur T_{calc} = 1300 °C (oben) und T_{calc} = 1450 °C (unten) verdeutlichen dies.

In den bei 1300 °C gesinterten Partikeln kann man die Mikroporosität in der Oberfläche noch erkennen. Hingegen sind die bei 1450°C kalzinierten γ-Al₂O₃-Partikel dicht.

Beide Varianten lassen sich nun mit sinteraktiven Tonerdepartikeln mit einer mittleren Korngröße von etwa 0,3 bis etwa 0,4 µm beschichten. Je nach Konzentration und Sintertemperatur werden dadurch die gesinterten Sekundärkörner zusammengesintert.

Hilfreich für das Sinterverhalten ist die Zulegierung von Sinterhilfsmitteln, wie beispielsweise SiO₂, MgO und/oder TiO_{2.}

Eine sehr gute und homogene Porosität wird im Sinterformkörper erreicht, wenn der Anteil an gesinterten Sekundärkörnern zwischen 75 bis 85 Gewichtsprozent und die Sinteradditive in einer Konzentration von 15 bis 25 Gewichtsprozent vorliegen. der Hauptbestandteil im Sinteradditiv ist Al₂O₃. Bezogen auf die Gesamtzusammensetzung liegt der Anteil an MgO bei maximal 1 Gewichtsprozent und der Anteil an TiO₂ bei maximal 2,5 Gewichtsprozent.

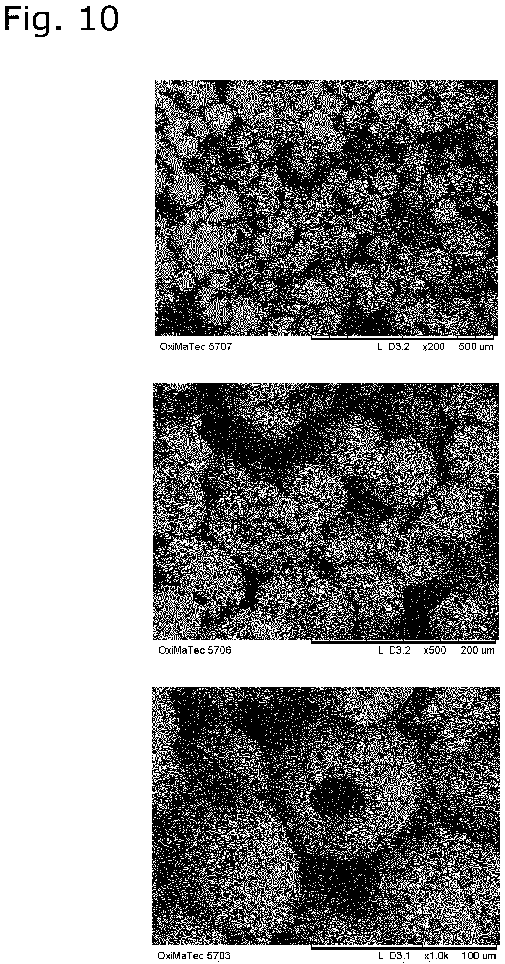

Die in Figur 10 dargestellten REM-Bruch-Bilder zeigen die Mikrostruktur der so hergestellten Dauerfilter.

Im Gegensatz zu Keramiken, die auf dem Einsatz von kommerziell erhältlichem Elektroschmelzkorund basieren, der mit Sinterhilfsmitteln versetzt, in Form gebracht und gesintert wird, zeigen Dauerfilter die wie beschrieben hergestellt werden wegen der fraktionierten Ausgangskörner und deren kugeliger Form eine sehr homogene Porenstruktur über das gesamte Filter. Bei Keramiken auf Basis von kommerziell erhältlichem Elektroschmelzkorund findet man neben feinen Poren häufig auch sehr grobe Poren, also insgesamt eine sehr inhomogene Porosität.

### Ausführungsbeispiel C:

Die Entwicklung der porösen Keramikfilter basiert im Wesentlichen darauf, dass zunächst γ-Al₂O₃ dispergiert und sprühgetrocknet wird. Nach der Fraktionierung durch Sieben des Sprühkorns wird dieses einer Kalzination unterzogen. In diesem Kalzinationsschritt wird das γ-Al₂O₃ in die thermodynamisch stabile α-Modifikation überführt. Damit verbunden ist ein Schwindungsprozess. Außerdem kann mit diesem Schritt - je nach Höhe der Kalzinationstemperatur - das Sekundärkorn in seinen Oberflächeneigenschaften entsprechend gestaltet werden. Die globuläre Struktur bleibt erhalten. Diese Sekundärkörner bilden die Matrix für die Herstellung des Filters. In den Versuchsreihen V0500 - V0504 konnte gezeigt werden, dass einerseits ein sinteraktives Aluminiumoxidpulver und zusätzlich die Zulegierung von geringen Mengen an Titanoxid und Magnesiumoxid hilfreich sind, um die Sintertemperatur zu senken beziehungsweise den Haftverbund zu verbessern. Damit lassen sich hohe Durchflussraten entsprechend dem Zielgebiet realisieren.

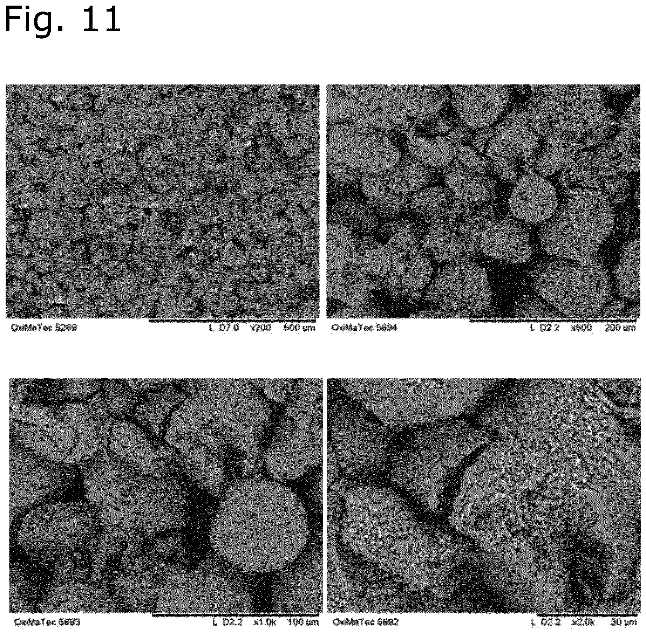

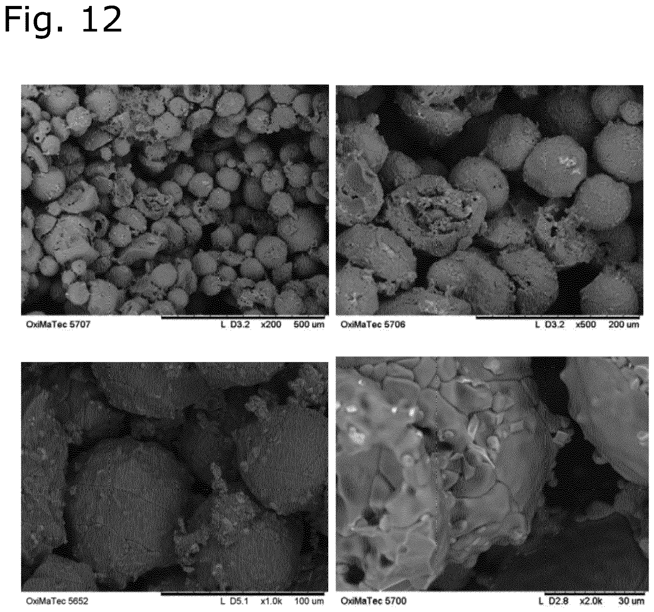

Nachfolgend werden REM-Untersuchungen an ausgewählten Sinterformkörpern näher beschrieben.
a) REM-Untersuchungen an V0500 - gesintert bei 1700°C/5h
   V0500 basiert auf der Sekundärkornfraktion 150 - 90 µm, hat außerdem 15 Gewichtsprozent APA0.5 und 0,25 Gewichtsprozent SiO₂ im Stoffversatz. Die Sekundärkornfraktion wurde bei 1350°C kalziniert. Für den Durchfluss wurden 87 ml/sec*bar*cm² gemessen. Figur 11 zeigt REM-Bilder an V0500.
b) REM-Untersuchungen an ersten Mustern aus dem Presswerkzeug
   Zunächst wurde die Kornfraktion 150 - 90 µm bei 1450°C kalziniert, dann mit 15 Gewichtsprozent APA0.5, 1,25 Gewichtsprozent TiO₂ und 0,2 Gewichtsprozent MgO versetzt und schließlich der Belegungsmahlung unterzogen. Abschließend wurde dann noch das Bindemittel hinzugefügt, bevor dieser Versatz im vorhandenen Preßwerkzeug in Form gebracht, entbindert und anschließend bei 1650°C für 3 Stunden gesintert wurde. Figur 12 zeigt entsprechende REM-Aufnahmen.

Durch die angewendete Kalzinationstemperatur kann die Stabilität der Sekundärkörner einerseits und die Oberflächenbeschaffenheit andererseits beeinflusst werden. Je niedriger die Kalzinationstemperatur für die Umwandlung von γ-Al₂O₃ nach α- Al₂O₃ gewählt wird, desto höher bleibt die Oberfläche auf den einzelnen Sekundärkörnern. Andererseits lassen sich niedriger gesinterte Sekundärkörner beim Pressen relativ leicht zerstören. Mit höherer Kalzinationstemperatur steigt die Stabilität der Sekundärkörner. Außerdem wird bei diesem Ansatz ein höherer Durchfluss erwartet. Für die Einstellung der Durchflussrate des Dauerfilters ergeben sich folgende Parameter:
- Siebfraktion der γ-Al₂O₃-Sekundärkörner,
- Kalzinationstemperatur für die γ-Al₂O₃ nach α- Al₂O₃-Phasenumwandlung,
- Zulegierung der Konzentration an sinteraktiver α- Tonerde,
- Zulegierung von Sinteradditiven,
- Sintertemperatur.

### Ausführungsbeispiel D:

Zur Herstellung eines Dauerfilters wurde eine γ-Tonerde mit entsprechender Kornverteilung aufbereitet, gesiebt und das Siebgranulat dann entsprechend kalziniert. Dabei sind dann zunächst definierte Fraktionen bei unterschiedlichen Temperaturen kalziniert und anschließend mit Tonerde versetzt worden, um die Sinteraktivität zu erhöhen. Außerdem sind diesen Versätzen als Sinterhilfsmitteln noch SiO₂ und MgO in den gemäß ISO 6474 erlaubten Mengen hinzu dotiert worden. In einer Reihe von Versuchsansätzen konnten die Durchflussraten gesteigert werden, und doch letztlich ist der in Aesculap gemessene Durchfluss mit ca. 30 ml/sec*bar*cm² noch zu niedrig.

Die Herstellung von Granulat aus γ-Al₂O₃erfolgte durch Mahlung. Nach dem Sprühtrocknen wurde das Granulat einer scharfen Fraktionierung unterzogen. Fokussiert wurde auf die Klassifizierungen
i) 150 - 106 µm und
ii) 106 - 90 µm.

Diese Fraktionen sind dann im Anschluss bei 1390 °C kalziniert worden.

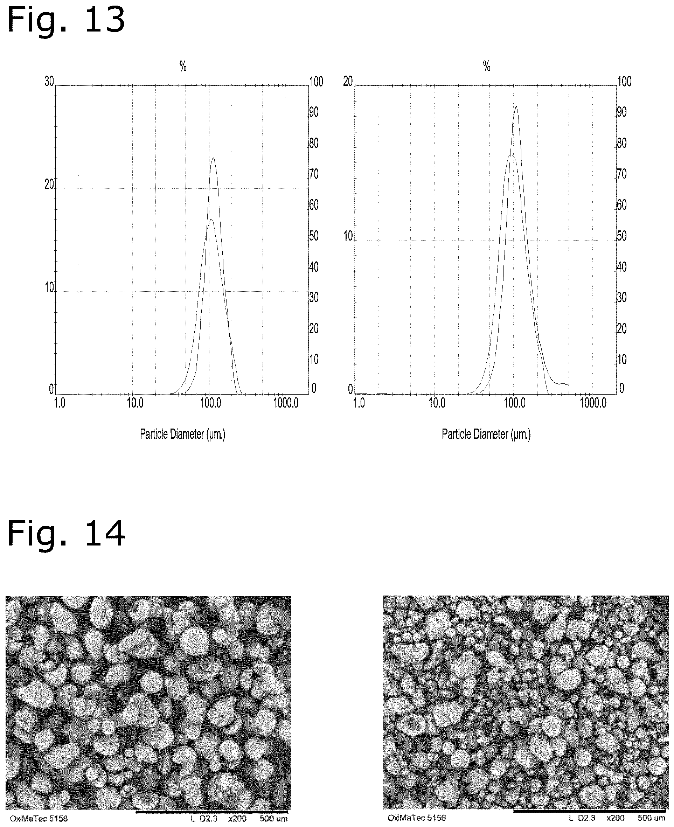

Da bei der Phasenumwandlung von γ- nach α-Al₂O₃ eine Schrumpfung stattfindet, wurde zunächst ermittelt, wie sich das Granulat in der Kornverteilung verhält. Figur 14 zeigt die Granulatverteilung der Variante i) (links: blau = γ-Al₂O₃, rot = α-Al₂O₃) und ii) (rechts: blau = γ-Al₂O₃, rot = α-Al₂O₃).

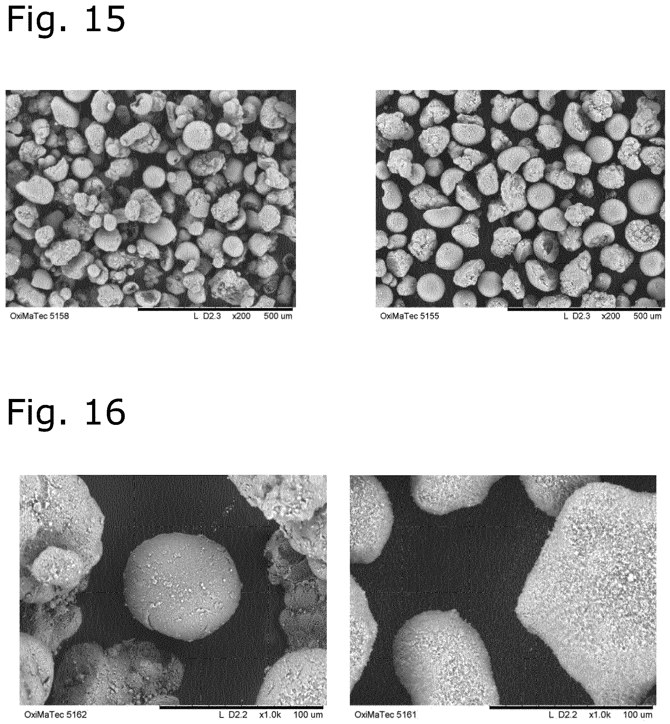

Tabelle 1 gibt die Kornverteilung wieder. Figur 15 zeigt die REM-Aufnahmen der Siebfraktion 150 - 90 µm; Figur 16 der Siebfraktion 106 - 90 µm.

**Tabelle 1: Kornverteilung der Siebfraktionen vor und nach Kalzination**

| | **Fraktion 150**-**90µm** | | **Fraktion 106 - 90 µm** | |
|---|---|---|---|---|
| | **┐-Al₂O3** | **┐-Al₂O₃** | **┐-Al₂O3** | **┐-Al₂O₃** |
| d₁₀ [µm] | 81 | 68 | 72 | 61 |
| d₅₀[µm] | 115 | 107 | 111 | 98 |
| d₉₀ [µm] | 164 | 172 | 192 | 164 |

Für die Herstellung einer homogenen Mischung kann die sinteraktive Tonerde in Wasser dispergiert und dann mit dem Kalzinat versetzt werden. Alternativ hierzu bietet sich ein Trockenverfahren an: Mischmahlung trocken mit 2 % Binder auf der Rollbank. Letzteres Verfahren hat sich als sehr effizient gezeigt, denn die sinteraktive Tonerde zieht sich dabei homogen auf die kalzinierten groben Körner auf. Abbildung 4 zeigt das reine Kalzinat (links) und das mit 15 Gewichtsprozent APA0.5 versetzte Kalzinat.

In den verschiedenen Versuchen zeigte sich, dass die Fraktion 106 - 90 µm nach dem Sintern mit Tonerde und weiteren Dopanden zu niedrigeren Durchflussraten führt, als dies für die Fraktion 150 - 90 µm der Fall ist. Vor diesem Hintergrund wurden die Versätze auf Basis von der letzteren Fraktion näher untersucht. Tabelle 2 fasst die Ergebnisse zusammen.

**Tabelle 2: Chemische Zusammensetzung, Durchflussraten und Porosität**

| **Versatz** | **Fraktion [µm]** | **Anteil APA0.5 [wt-%]** | **Dotierung** | **Sintem [°C/h]** | **Porosität [%]** | **Durchfluß OxiMaTec [ml/sec*bar*cm²]** | **Durchfluß Aesculap [ml/sec*bar*cm²]** | **Stabilität** |
|---|---|---|---|---|---|---|---|---|
| | 106-90 | 15 | 0,25 % SiO₂ | 1700/5 | 32 | 250 | | nicht schlecht |
| V0500 | 150-90 | 15 | 0,25 % SiO₂ | 1600/5 | | 45 | - | ungenügend |
| V0500 | 150-90 | 15 | 0,25 % SiO₂ | 1700/5 | 33 | 360 | 87 | besser |
| V0501 | 150-90 | 15 | - | 1600/5 | | 45 | | ungenügend |
| V0501 | 150-90 | 15 | - | 1700/5 | 29 | 290 | 105 | nicht schlecht |
| V0502 | 150-90 | 15 | 0,25 % TiO₂ | 1600/5 | | 400 | | mangelhaft |
| V0502 | 150-90 | 15 | 0,25 % TiO₂ | 1700/5 | 35 | 480 | 71 | besser |
| V0503 | 150-90 | 15 | 1,25 % TiO₂ | 1600/5 | | 350 | | besser |
| V0503 | 150-90 | 15 | 1,25 % TiO₂ | 1700/5 | 31 | 590 | 84 | nicht schlecht |
| V0504 | 150-90 | 15 | 0,25 % TiO₂ + 0,2%MgO | 1700/5 | | 5 | 54 | sehr gut |
| V0504 | 150-90 | 15 | 0,25 % TiO₂ + 0,2 % MgO | 1525/5 | | 400 | | gut |

Hohe Durchflussraten bei guter Sinterkörperstabilität werden bei hohen Sintertemperaturen erreicht. Die Dotierung mit MgO und TiO₂ in geringen Konzentrationen führt zu einer enormen Steigerung der Sinteraktivität bei gleichzeitig hoher Luftdurchlässigkeit.

Nach den vorliegenden Ergebnissen eignet sich eine breitere Verteilung der Poren im groben Bereich besser, als eine enge. Mit der Siebfraktion 150 - 90 µm lassen sich Durchflussraten erzielen, welche im Zielgebiet liegen. Eine sehr gute Stabilität der Sinterformkörper erreicht man, wenn der Versatz mit Titandioxid und Magnesiumoxid dotiert wird. Durch diese Maßnahme lässt sich auch die Sintertemperatur senken. Versätze ohne Dotierung benötigen eine sehr hohe Sintertemperatur um hohe Durchflussraten und eine gute Stabilität der Formkörper zu erzielen.

Mit den vorliegenden Versuchsergebnissen konnte eine gewünschte Durchflussrate erzeugt werden. Es erscheint sinnvoll, parallel zur verfahrenstechnischen Umsetzung in einen Spritzgussversatz weitere Detailoptimierungen mit TiO₂- und MgO-Konzentrationsvariationen durchzuführen. Damit lässt sich unter Umständen die Sintertemperatur weiter senken.

### Ausführungsbeispiel E:

Auch hier wurden Aluminiumoxidagglomerate über γ-Al₂O₃ herstellt und diese mit Tonerde bzw. zusätzlichen Additiven gesintert.

Zwecks Realisierung der gewünschten Durchflussmenge im Filter hat sich gezeigt, dass die über den Sprühtrocknungsprozess erzeugten Agglomerate aus der γ-Tonerde vorzugsweise in einer speziellen Fraktion vorliegen sollten. Vor diesem Hintergrund wurde ein Pilotansatz mit γ-Al₂O₃ in der RWK gemahlen, mit Bindemittel versetzt und dann in einer sehr breiten Kornverteilung sprühgetrocknet. Nach der Sprühtrocknung sind die folgenden Siebfraktionen herge-stellt worden:
- > 150 µm
- 150 - 106 µm
- 106 - 63 µm
- 63 - 45 µm
- < 45 µm

Die einzelnen Kornfraktionen wurden dann teilweise bei 1050 °C und bei 1350 °C kalziniert und damit in α-Al₂O₃ überführt.

Sämtliche Stoffansätze zeigen einen Luftdurchfluss; die Durchflussrate ist jedoch in verschiedenen Proben sehr gering und mit der vorhandenen Meßapparatur nicht messbar.

Die höchsten Durchflussraten erhält man, wenn man die Fraktion 106 - 63 µm mit Tonerde APA0.5 beziehungsweise zusätzlich mit SiO₂ und/oder MgO im Rahmen der zulässigen Werte gemäß ISO6474-Teil 1 einsetzt. Über die Konzentration der als Sinterhilfsmittel eingesetzten Tonerde kann die Durchfluss-rate in gewissen Grenzen beeinflusst werden. Für die Abriebfestigkeit ist hauptsächlich die angewendete Sintertemperatur verantwortlich. Bei Sintertemperaturen von 1525 °C und mehr wird die Stabilität des Kornverbundes immer besser.

Nachfolgende Tabelle gibt einen Überblick zu den durchgeführten Stoffansätzen und deren Verhalten während des Sinterns bzw. das Durchflussverhalten nach dem Sintern:

| | **Kornfraktion [µm]** | **Kalzinations-temperatur [°C]** | **Sintern** | **Ansatz** | **ρ [g/cm³]** | **P [%]** | **Durchfluß [ml/sec*bar*cm²]** |
|---|---|---|---|---|---|---|---|
| | | | 1390°C/5h | pur | 2,10 | 47 | "Blasen" |
| γ-Al₂O₃ | < 45 µm | - | 1450°C/5h | | 2,43 | 39 | "Blasen" |
| | | | 1525°C/5h | | 2,62 | 34 | "Blasen" |
| | | | 1390°C/5h | pur | 2,05 | 49 | "Blasen" |
| γ-Al₂O₃ | 63 - 45 µm | - | 1450°C/5h | | 2,38 | 40 | "Blasen" |
| | | | 1525°C/5h | | 2,54 | 36 | "Blasen" |
| | | | 1390°C/5h | pur | 2,35 | 41 | "Blasen" |
| γ-Al₂O₃ | 106 - 63 µm | - | 1450°C/5h | | 2,74 | 31 | "Blasen" |
| | | | 1525°C/5h | | 2,81 | 30 | "Blasen" |
| | | | 1390°C/5h | 75 wt-% γ-Al₂O₃ + 25 wt-% APA0.5 | 2,39 | 40 | "Blasen" |
| V0451 | > 150 µm | 1050 °C | 1450°C/5h | | 2,74 | 31 | "Blasen" |
| | | | 1525°C/5h | | 2,95 | 26 | "Blasen" |
| | | | 1390°C/5h | 75 wt-% γ-Al₂O₃ + 25 wt-% APA0.5 | 2,32 | 42 | "Blasen" |
| V0452 | 150 - 106 µm | 1050 °C | 1450°C/5h | | 2,70 | 32 | "Blasen" |
| | | | 1525°C/5h | | 2,89 | 28 | "Blasen" |
| | | | 1390°C/5h | 75 wt-% γ-Al₂O₃ + 25 wt-% APA0.5 | 2,30 | 42 | "Blasen" |
| V0453 | 106 - 63 µm | 1050 °C | 1450°C/5h | | 2,66 | 33 | "Blasen" |
| | | | 1525°C/5h | | 2,85 | 29 | "Blasen" |
| | | | 1390°C/5h | 75 wt-% γ-Al₂O₃ + 25 wt-% APA0.5 | 2,37 | 41 | "Blasen" |
| V0454 | 63 - 45 µm | 1050 °C | 1450°C/5h | | 2,71 | 32 | "Blasen" |
| | | | 1525°C/5h | | 2,90 | 27 | "Blasen" |
| | | | 1390°C/5h | 75 wt-% γ-Al₂O₃ + 25 wt-% APA0.5 | 2,40 | 40 | "Blasen" |
| V0455 | < 45 µm | 1050 °C | 1450°C/5h | | 2,77 | 31 | "Blasen" |
| | | | 1525°C/5h | | 2,97 | 26 | "Blasen" |
| | | | 1390°C/5h | 75 wt-% γ-Al₂O₃ + 25 wt-% APA0.5 | 2,34 | 41 | 33 |
| V0456 | 150 - 106 µm | 1350 °C | 1450°C/5h | | 2,53 | 37 | 13 |
| | | | 1525°C/5h | | 2,68 | 33 | 13 |
| | | | 1580°C/5h | | 2,71 | 32 | 40 |
| | | | 1390°C/5h | 85 wt-% γ-Al₂O₃ + 15 wt-% APA0.5 | 2,16 | 46 | 53 |
| V0457 | 150 - 106 µm | 1350 °C | 1450°C/5h | | 2,36 | 41 | 80 |
| | | | 1525°C/5h | | 2,46 | 38 | 87 |
| | | | 1580°C/5h | | 2,51 | 37 | 67 |
| | | | 1390°C/5h | 75 wt-% γ-Al₂O₃ + 25 wt-% APA0.5 | 2,13 | 47 | 67 |
| V0458 | 106 - 63 µm | 1350 °C | 1450°C/5h | | 2,43 | 39 | 67 |
| | | | 1525°C/5h | | 2,44 | 39 | 60 |
| | | | 1580°C/5h | | 2,29 | 43 | 147 |
| | | | 1390°C/5h | 85 wt-% γ-Al₂O₃ + 15 wt-% APA0.5 | 1,96 | 51 | 153 |
| V0459 | 106 - 63 µm | 1350 °C | 1450°C/5h | | 2,17 | 46 | 167 |
| | | | 1525°C/5h | | 2,24 | 44 | 200 |
| | | | 1580°C/5h | | 2,29 | 43 | 180 |
| | | | 1390°C/5h | 85 wt- % g-Al₂O₃ + 15wt-% APA0.5 | 2,07 | 48 | "Blasen" |
| V0460 | 63 - 45 µm | 1350 °C | 1450°C/5h | | 2,27 | 43 | 47 |
| | | | 1525°C/5h | | 2,37 | 41 | 33 |
| | | | 1390°C/5h | 85 wt- % g-Al2O3 + 15wt-% APA0.5 | 2,25 | 44 | "Blasen" |
| V0461 | < 45 µm | 1350 °C | 1450°C/5h | | 2,30 | 42 | "Blasen" |
| | | | 1525°C/5h | | 2,66 | 33 | "Blasen" |
| V0462 | 106 - 63 µm | 1350 °C | 1390°C/5h | 80 wt-%g-Al2O3 + 20 wt-% APA0.5 | 1,94 | 51 | 100 |
| | | | 1525°C/5h | | 2,22 | 44 | 107 |
| V0465 | 63 - 45 µm | 1350 °C | 1390°C/5h | 80 wt-% g-Al2O3 + 20wt-% APA0.5 | 2,26 | 43 | "Blasen" |
| | | | 1525°C/5h | | 2,59 | 35 | "Blasen" |
| V0463 | 106 - 63 µm | 1350 °C | 1390°C/5h | 80 wt- % g-Al2O3 + 19,95 wt-% APA0.5 + 0,05 w-% SiO₂ | 1,94 | 51 | 133 |
| | | | 1525°C/5h | | 2,20 | 45 | 160 |
| V0466 | 63 - 45 µm | 1350 °C | 1390°C/5h | 80 wt-% g-Al2O3 + 19,95wt-% APA0.5 + 0,05 w-% SiO₂ | 2,06 | 48 | "Blasen" |
| | | | 1525°C/5h | | 2,40 | 40 | "Blasen" |
| V0464 | 106 - 63 µm | 1350 °C | 1390°C/5h | 80wt-% g-Al2O3+ 19,95 wt-% APA0.5 + 0,05 w-% SiO₂+ 0,04 wt-% MgO | 2,06 | 48 | 127 |
| | | | 1525°C/5h | | 2,38 | 40 | 113 |
| V0467 | 63 - 45 µm | 1350 °C | 1390°C/5h | 80 wt-%g-Al2O3 + 19,95 wt-% APA0.5 + 0,05w-% SiO₂ + 0,04 wt-% MgO | 2,10 | 47 | "Blasen" |
| | | | 1525°C/5h | | 2,46 | 38 | "Blasen" |
| | | | 1450°C/5h | 80 wt-% g-Al2O3 + 19,95 wt-% APA0.5 + 0,2w-% SiO2 + 0,2 wt % MgO, | 2,24 | 44 | 27 |
| V0480 | 106 - 63 µm | 1350 °C | 1525°C/5h | | 2,36 | 41 | 127 |
| | | | 1600°C/5h | | 2,43 | 39 | 120 |
| V0481 | 63 - 45 µm | 1350 °C | 1525°C/5h | 80 wt-% g-Al2O3 + 19,95 wt-% APA0.5 + 0,2w-% SiO2 | 2,41 | 39 | "Blasen" |
| | | | 1600°C/5h | | 2,63 | 34 | "Blasen" |
| V0482 | 63 - 45 µm | 1350 °C | 1525°C/5h | 80 wt-%g-Al2O3 + 19,95 wt-% APA0.5 + 0,2w-% SiO2 + 0,2 wt % MgO | 2,57 | 35 | "Blasen" |
| | | | 1600°C/5h | | 2,63 | 34 | "Blasen" |

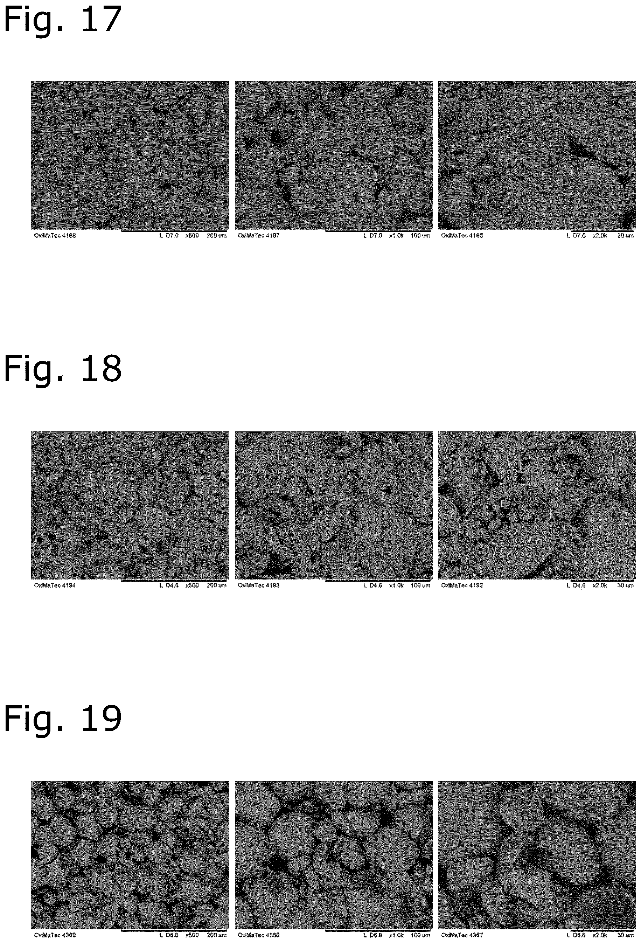

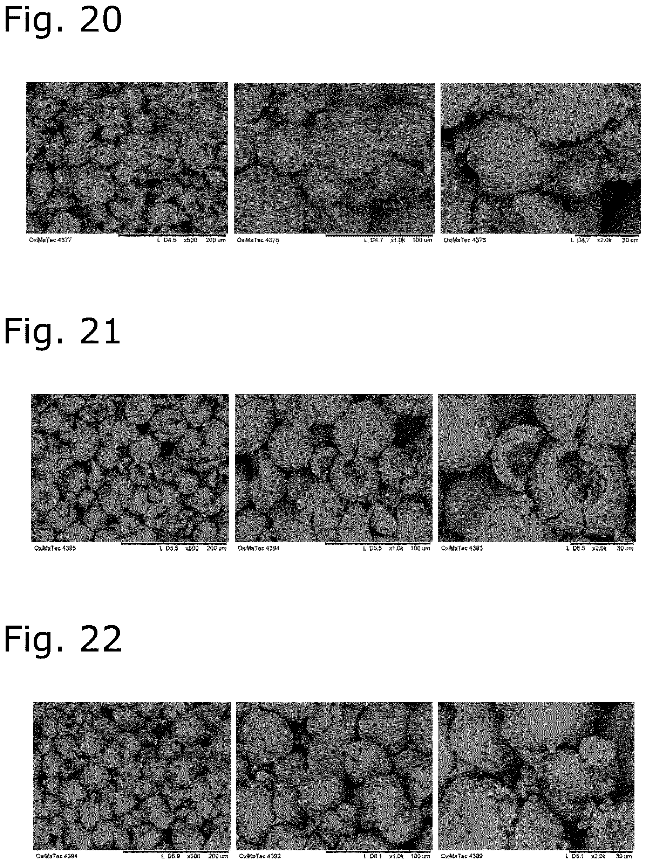

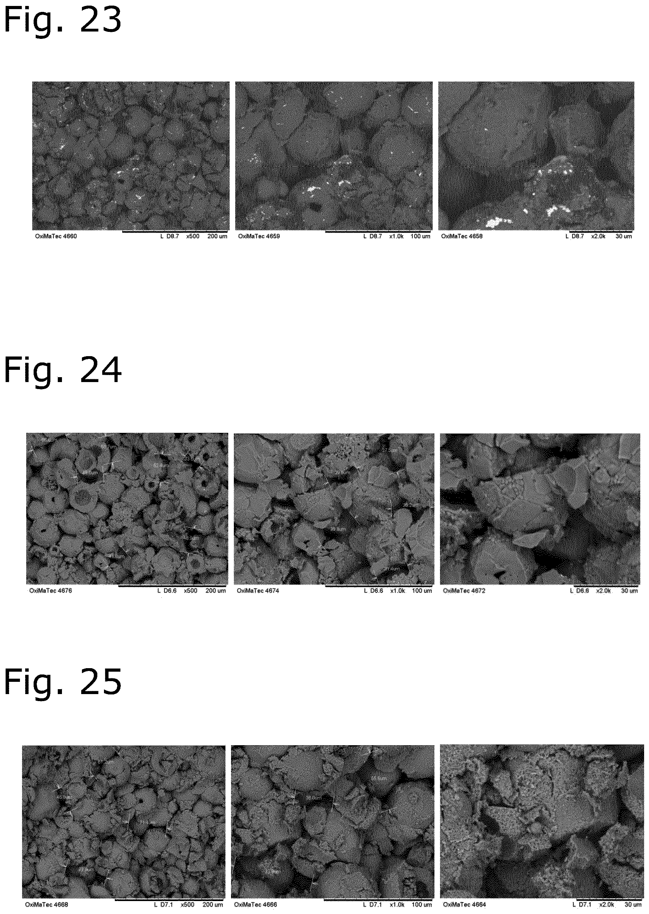

Ausgewählte REM-Bilder zu den durchgeführten Stoffansätzen sind in den nachfolgenden Figuren 17 bis 25 dargestellt:
In den durchgeführten Versuchen konnte bei Verwendung der Fraktion 106 - 63 µm stets eine hohe Durchflussrate erzielt werden. Je höher die Sintertemperatur wird, desto mehr verschwindet das feinkörnige Gefüge.

Andererseits zeigen die Bauteile mit hoher Sintertemperatur Poren innerhalb der Hohlkörner, welche im Sprühprozess erzeugt werden.

Mit zunehmender Sintertemperatur steigt auch die Abriebfestigkeit. In den durchgeführten Versuchen konnte kein signifikanter Einfluss der Dotierung durch SiO₂ beziehungsweise MgO festgestellt werden. Tendenziell scheinen die dotierten Körper eine etwas höhere Stabilität aufzuweisen.

Aus den REM-Aufnahmen wird auch ersichtlich, dass reines γ-Al₂O₃ ebenso wenig für einen hohen Durchfluss geeignet ist, als eine lediglich bei 1050°C kalzinierte Tonerde. Dies hängt damit zusammen, dass die Sekundärkörner bei der Formgebung zerdrückt und damit die Porenkanäle verengt werden. Besonders deutlich ist dies in den Bruchbildern der Abbildung 18 zu sehen.

### Optionale Hydrophobierung der Dauerfilter:

Ferner können die oben beschriebenen Dauerfilter optional mit einer Siloxan-beschichtung versehen werden, die ohne Beschichtung stark ausgeprägte Hydrophilie der Dauerfilter deutlich zu reduzieren.

Die Siloxanbeschichtung wird erzeugt, indem auf einen neu hergestellten Dauerfilter eine Siloxan-Schicht aufgebracht wird. Diese kann optional bei einer Temperaturen von mindestens 150°C getempert, quasi "eingebrannt" werden.

Auf diese Weise beschichtete keramische Dauerfilter zeigen beim Wassereindringtest mit Fuchsinlösung eine sehr gute Hydrophobie.

Untersuchungen zur Korrosionsbeständigkeit der Siloxanbeschichtung ergaben eine gute Resistenz im alkalischen Bereich bis zu einem pH-Wert von etwa 10. Bei hoch alkalischen Reinigungsmitteln, wie sie zum Reinigen der Filter vor der Sterilisation eingesetzt werden, zeigt die Schicht bereits nach einer Behandlung von etwa 1 Stunde allerdings deutliche Korrosionserscheinungen.

Obwohl die Hydrophobierungsschicht aus Siloxan extrem dünn ist, beobachtet man im Luftdurchfluss bereits einen geringeren Volumenstrom, als in den nicht beschichteten Filtern.

Unter Berücksichtigung der Korrosionsprobleme von Siloxanbeschichtungen im hoch alkalischen Bereich, die zu einer Abnahme der hydrophobierenden Wirkung im Lauf der Zeit führen kann, wurde alternativ eine hydrophobe Beschichtung vorgeschlagen, die Teflon enthält. Teflon ist als äußerst korrosionsbeständiger Werkstoff bekannt.

Zur Beschichtung neuer keramischer Dauerfilter mit Teflon wird ein mikronisiertes Teflonpulver eingesetzt. Dieses lässt sich allerdings nur relativ schwierig dispergieren und kann lediglich an der Oberfläche der Filter abgeschieden werden. Allerdings haftet diese Beschichtung sehr gut auf der Oberfläche der Filter an und ist gegenüber insbesondere alkalischen Reinigungsmitteln sehr widerstandsfähig, auch mit einem pH-Wert größer als 10.

Alternativ zum beschriebenen mikronisierten Teflonpulver kann zur Beschichtung auch eine Teflonemulsion eingesetzt werden, welche zur Beschichtung von Textilien eingesetzt wird. Es kann so ebenfalls eine hydrophobe Beschichtung der Dauerfilter ausgebildet werden. Insbesondere ermöglicht es die Teflonemulsion, eine Hydrophobierung der Dauerfilter im Volumen zu erreichen. Dabei ist zu beobachten, dass mit steigender Konzentration der Teflonemulsion die Luftdurchlässigkeit nachlässt. An zylindrischen Proben von Dauerfiltern wurde die Durchlässigkeit untersucht. Es ergaben sich folgende Werte:

| Verdünnung der Teflonemulsion | Durchflussrate |
|---|---|
| 1:50 | 35 ml/s · bar · cm² |
| 1:10 | 25 ml/s · bar · cm² |
| 1:5 | 20 ml/s · bar · cm² |

Durch die in der beschriebenen Weise aufgebrachte Teflonbeschichtung konnte auch nach 2 h Kochen in Reinigungslösung keine Korrosion festgestellt werden. Allerdings verringert die Teflonbeschichtung den Luftdurchfluss durch den Dauerfilter im Vergleich zum unbeschichteten Filter. Je höher die Konzentration der Teflonsuspension, desto stärker wird der Luftdurchfluss reduziert.

Um den gewünschten Durchfluss auch bei mit einer Teflonbeschichtung versehenen Dauerfilter zu erreichen, wird vorzugsweise eine gröbere Porenstruktur für den Dauerfilter gewählt, bevor er mit einer Teflonbeschichtung hydrophobiert wird. So kann insbesondere ein wünschenswerter Durchfluss von mindestens 100 ml/s · bar · cm² erreicht werden.

## Patentansprüche

1. Dauerfilter (22; 22') für einen medizinischen Sterilisationsbehälter (10), wobei der Dauerfilter (22; 22') aus einer Keramik hergestellt ist und dass die Keramik aus globulären Substratkörnern hergestellt ist **dadurch gekennzeichnet, dass** der Dauerfilter (22; 22') mit einer hydrophoben Beschichtung versehen ist und dass die hydrophobe Beschichtung im Volumen des Dauerfilters (22; 22') aufgebracht ist.

2. Dauerfilter nach Anspruch 1, **dadurch gekennzeichnet, dass** der Dauerfilter (22; 22') selbsttragend ohne Trägerelement ausgebildet ist.

3. Dauerfilter nach Anspruch 1 oder 2, **dadurch gekennzeichnet, dass** die Substratkörner hergestellt sind durch
• Dispergieren und De-Agglomerieren von Keramikpulver in wässriger Suspension zur Erzeugung einzelner Primärkörner,
• Sprühtrocknen der die Primärkörner enthaltenden Suspension und
• Kalzinieren der Primärkörner zu Sekundärkörnern, welche die globulären Substratkörner bilden.

4. Dauerfilter nach Anspruch 3, **dadurch gekennzeichnet, dass** die globulären Substratkörner durch das Kalzinieren von γ-Aluminiumoxid (y-Al₂O₃) in α-Aluminiumoxid (α-Al₂O₃) hergestellt sind.

5. Dauerfilter nach einem der voranstehenden Ansprüche, **dadurch gekennzeichnet, dass** der Dauerfilter (22; 22') hergestellt ist durch Zusetzen von Sinteradditiven zu den globulären Substratkörnern vor dem Sintern.

6. Dauerfilter nach einem der Ansprüche 3 bis 5, **dadurch gekennzeichnet, dass** der Dauerfilter (22; 22') hergestellt ist durch Sprühtrocknen der die Primärkörner enthaltenden Suspension unter Zugabe eines organischen Bindemittels, insbesondere von Polyvinylalkohol oder Polyacrylat.

7. Medizinischer Sterilisationsbehälter (10), insbesondere zum Aufnehmen und Lagern zu sterilisierender Gegenstände, mit einem Behälterunterteil (12) und einem Behälteroberteil (14) zum Verschließen des Behälterunterteils (12) in einer Schließstellung des Sterilisationsbehälters (10), wobei der Sterilisationsbehälter (10) einen Behälterinnenraum (16) definiert, welcher vom Behälterunterteil (12) und vom Behälteroberteil (14) begrenzt wird, und wobei der Behälterunterteil und/oder der Behälteroberteil eine Gasaustauschöffnung (26) aufweisen, welche mit einem Dauerfilter (22; 22') verschlossen ist, **gekennzeichnet durch** einen Dauerfilter (22; 22') nach einem der voranstehenden Ansprüche.

8. Verfahren zum Herstellen eines Dauerfilters (22; 22') für einen medizinischen Sterilisationsbehälter (10), bei welchem der Dauerfilter (22, 22') durch Sintern aus einem keramischen Material hergestellt wird und als keramisches Material globuläre Substratkörner verwendet werden **dadurch gekennzeichnet, dass** der Dauerfilter (22; 22') mit einer hydrophoben Beschichtung versehen wird und dass die hydrophobe Beschichtung im Volumen des Dauerfilters (22; 22') aufgebracht wird.

9. Verfahren nach Anspruch 8, **dadurch gekennzeichnet, dass** die Substratkörner hergestellt werden durch
• Dispergieren und De-Agglomerieren von Keramikpulver in wässriger Suspension zur Erzeugung einzelner Primärkörner,
• Sprühtrocknen der die Primärkörner enthaltenden Suspension und
• Kalzinieren der Primärkörner zu Sekundärkörnern, welche die globulären Substratkörner bilden.

10. Verfahren nach Anspruch 9, **dadurch gekennzeichnet, dass** durch das Kalzinieren γ-Aluminiumoxid (γ-Al₂O₃) in α-Aluminiumoxid (α-Al₂O₃) umgewandelt wird.

11. Verfahren nach einem der Ansprüche 8 bis 10, **dadurch gekennzeichnet, dass** zur Ausbildung des Dauerfilters (22; 22') globuläre Substratkörner bei Temperaturen im Bereich von etwa 1350°C bis etwa 1700°C, vorzugsweise bei etwa 1390°C bis etwa 1650°C, gesintert werden.

12. Verfahren nach einem der Ansprüche 8 bis 11, **dadurch gekennzeichnet, dass** den globulären Substratkörnern vor dem Sintern Sinteradditive zugesetzt werden.

13. Verfahren nach einem der Ansprüche 9 bis 12, **dadurch gekennzeichnet, dass** eine Fraktionierung der sprühgetrockneten Primärkörner durch Sieben erfolgt.

14. Verfahren nach einem der Ansprüche 9 bis 13, **dadurch gekennzeichnet, dass** das Sprühtrocknen der Suspension unter Zugabe eines organischen Bindemittels, insbesondere von Polyvinylalkohol oder Polyacrylat, erfolgt.

15. Verfahren nach einem der Ansprüche 8 bis 14, **dadurch gekennzeichnet, dass** der Dauerfilter (22; 22') selbsttragend ohne Trägerelement hergestellt wird.

## Claims

1. Permanent filter (22; 22') for a medical sterilization container (10), wherein the permanent filter (22; 22') is made from a ceramic and wherein the ceramic is made from globular substrate grains, **characterized in that** the permanent filter (22; 22') is provided with a hydrophobic coating and **in that** the hydrophobic coating is applied over the entire volume of the permanent filter (22; 22').

2. Permanent filter according to claim 1, wherein the permanent filter (22; 22') is self-supporting, without a support element.

3. Permanent filter according to claim 1 or 2, **characterized in that** the substrate grains are produced by
• dispersing and deagglomerating ceramic powder in aqueous suspension to produce individual primary grains,
• spray drying the suspension containing the primary grains and
• calcining the primary grains to yield secondary grains, which form the globular substrate grains.

4. Permanent filter according to claim 3, **characterized in that** the globular substrate grains are produced by calcining γ-aluminium oxide (γ-Al₂O₃) into α-aluminium oxide (α-Al₂O₃).

5. Permanent filter according to any one of the preceding claims, **characterized in that** the permanent filter (22; 22') is produced by adding sintering additives to the globular substrate grains prior to sintering.

6. Permanent filter according to any one of claims 3 to 5, **characterized in that** the permanent filter (22; 22') is produced by spray drying the suspension containing the primary grains with the addition of an organic binder, in particular of polyvinyl alcohol or polyacrylate.

7. Medical sterilization container (10), in particular for receiving and storing objects to be sterilized, with a container bottom part (12) and a container top part (14) for closing the container bottom part (12) in a closed position of the sterilization container (10), the sterilization container (10) defining a container interior (16), which is delimited by the container bottom part (12) and by the container top part (14), and the container bottom part and/or the container top part having a gas exchange orifice (26), which is closed with a permanent filter (22; 22'), **characterized by** a permanent filter (22; 22') according to any one of the preceding claims.

8. Method for producing a permanent filter (22; 22') for a medical sterilization container (10), **characterized in that** the permanent filter (22, 22') is produced from a ceramic material by sintering and **in that** globular substrate grains are used as the ceramic material, **characterized in that** the permanent filter (22; 22') is provided with a hydrophobic coating and **in that** said hydrophobic coating is applied over the entire volume of the permanent filter (22; 22').

9. Method according to claim 8, **characterized in that** the substrate grains are produced by
• dispersing and deagglomerating ceramic powder in aqueous suspension to produce individual primary grains,
• spray drying the suspension containing the primary grains and
• calcining the primary grains to yield secondary grains, which form the globular substrate grains.

10. Method according to claim 9, **characterized in that** γ-aluminium oxide (y-Al₂O₃) is transformed into α-aluminium oxide (α-Al₂O₃) by calcination.

11. Method according to any one of claims 8 to 10, **characterized in that** the permanent filter (22; 22') is formed by sintering globular substrate grains at temperatures in the range from around 1350°C to around 1700°C, preferably at around 1390°C to around 1650°C.

12. Method according to any one of claims 8 to 11, **characterized in that** sintering additives are added to the globular substrate grains prior to sintering.

13. Method according to any one of claims 9 to 12, **characterized in that** fractionation of the spray-dried primary grains proceeds by screening.

14. Method according to any one of claims 9 to 13, **characterized in that** spray drying of the suspension proceeds with the addition of an organic binder, in particular of polyvinyl alcohol or polyacrylate.

15. Method according to any one of claims 8 to 14, **characterized in that** the permanent filter (22; 22') is self-supporting, without a support element.

## Revendications

1. Filtre permanent (22, 22') pour un récipient ou contenant de stérilisation médical (10), le filtre permanent (22, 22') étant fabriqué en une céramique et la céramique étant fabriquée en grains de substrat globulaires, **caractérisé en ce que** le filtre permanent (22, 22') est pourvu d'un revêtement hydrophobe, et **en ce que** le revêtement hydrophobe est appliqué dans le volume du filtre permanent (22, 22').

2. Filtre permanent selon la revendication 1, **caractérisé en ce que** le filtre permanent (22, 22') est réalisé autoporteur, sans élément porteur.

3. Filtre permanent selon la revendication 1 ou 2, **caractérisé en ce que** les grains de substrat sont fabriqués par
• dispersion et dé-agglomération de poudre de céramique en suspension aqueuse, pour produire des grains primaires individuels,
• séchage par pulvérisation de la suspension renfermant les grains primaires, et
• calcination des grains primaires en grains secondaires, qui forment les grains de substrat globulaires.

4. Filtre permanent selon la revendication 3, **caractérisé en ce que** les grains de substrat globulaires sont fabriqués par la calcination d'oxyde d'aluminium γ (γ-Al₂O₃) en oxyde d'aluminium α (α-Al₂O₃).

5. Filtre permanent selon l'une des revendications précédentes, **caractérisé en ce que** le filtre permanent (22, 22') est fabriqué par l'addition d'additifs de frittage aux grains de substrat globulaires, avant le frittage.

6. Filtre permanent selon l'une des revendications 3 à 5, **caractérisé en ce que** le filtre permanent (22, 22') est fabriqué par séchage par pulvérisation de la suspension renfermant les grains primaires tout en additionnant un liant organique, notamment un alcool polyvinylique ou un polyacrylate.

7. Récipient ou contenant de stérilisation médical (10), notamment destiné à recevoir et à stocker des objets à stériliser, comprenant une partie inférieure de récipient (12) et une partie supérieure de récipient (14) pour fermer la partie inférieure de récipient (12) dans une position de fermeture du récipient de stérilisation (10), le récipient de stérilisation (10) définissant une chambre intérieure de récipient (16), qui est délimitée par la partie inférieure de récipient (12) et par la partie supérieure de récipient (14), et la partie inférieure de récipient et/ou la partie supérieure de récipient présentant une ouverture d'échange de gaz (26), qui est fermée par un filtre permanent (22, 22'), **caractérisé par** un filtre permanent (22, 22') selon l'une des revendications précédentes.

8. Procédé de fabrication d'un filtre permanent (22, 22') pour un contenant ou récipient de stérilisation médical (10), d'après lequel le filtre permanent (22, 22') est fabriqué par frittage, en un matériau céramique, et l'on utilise en guise de matériau céramique, des grains de substrat globulaires, **caractérisé en ce que** l'on munit le filtre permanent (22, 22') d'un revêtement hydrophobe, et **en ce que** l'on applique le revêtement hydrophobe dans le volume du filtre permanent (22, 22').

9. Procédé selon la revendication 8, **caractérisé en ce que** l'on fabrique les grains de substrat par
• dispersion et dé-agglomération de poudre de céramique en suspension aqueuse, pour produire des grains primaires individuels,
• séchage par pulvérisation de la suspension renfermant les grains primaires, et
• calcination des grains primaires en grains secondaires, qui forment les grains de substrat globulaires.

10. Procédé selon la revendication 9, **caractérisé en ce que** par la calcination, de l'oxyde d'aluminium γ (γ-Al₂O₃) est transformée en oxyde d'aluminium α (α-Al₂O₃).

11. Procédé selon l'une des revendications 8 à 10, **caractérisé en ce que** pour former le filtre permanent (22, 22'), on procède à un frittage de grains de substrat globulaires, à des températures situées dans une plage d'environ 1350°C à environ 1700°C, de préférence d'environ 1390°C à environ 1650°C.

12. Procédé selon l'une des revendications 8 à 11, **caractérisé en ce qu'**avant le frittage, on ajoute des additifs de frittage aux grains de substrat globulaires.

13. Procédé selon l'une des revendications 9 à 12, **caractérisé en ce qu'**un fractionnement des grains primaires séchés par pulvérisation, est effectué par tamisage.

14. Procédé selon l'une des revendications 9 à 13, **caractérisé en ce que** le séchage par pulvérisation de la suspension est effectué tout en additionnant un liant organique, notamment un alcool polyvinylique ou un polyacrylate.

15. Procédé selon l'une des revendications 8 à 14, **caractérisé en ce que** le filtre permanent (22, 22') est fabriqué de manière autoportante, sans élément de support.
